# EUROPEAN PATENT SPECIFICATION

(11) **EP 3 870 175 B1**
(45) Date of publication and mention of the grant of the patent: **28.08.2024**
(21) Application number: 19816443.6
(22) Date of filing: 25.10.2019
(51) Int. Cl.: A61K 9/00, A61K 31/502, A61P 1/16

(54) **ORAL AMINODIHYDROPHTHALAZINEDIONE COMPOSITIONS AND THEIR USE THE TREATMENT OF NON-VIRAL HEPATITIS**
ORALE AMINODIHYDROPHTHALAZINEDIONZUSAMMENSETZUNGEN UND DEREN VERWENDUNG ZUR BEHANDLUNG VON NICHTVIRALER HEPATITIS
COMPSITIONS ORALES D'AMINODIHYDROPHTHALAZINEDIONE ET LEUR UTILISATION DANS LE TRAITEMENT DE L'HEPATITE NON-VIRALE

(30) Priority: 26.10.2018 US 201862751260 P
(43) Date of publication of application: 01.09.2021
(73) Proprietor: IMMUNOPHARMA PLUS D.O.O., 1000 Ljubljana (SI)
(72) Inventor: ABIDOV, Musa, T., Moscow, 119607 (RU)
(74) Representative: Hoffmann Eitle
(86) International application number: PCT/IB2019/001148
(87) International publication number: WO 2020/084348

(56) References cited:
- WO-A1-2017/117586
- WO-A1-2017/140422
- US-A- 5 543 410
- US-A1- 2002 198 208
- US-A1- 2003 195 183
- US-A1- 2012 329 801
- US-B2- 8 536 171
- JUKIC: "etrahydrophthalazine derivative "sodium nucleinate" exert its anti-inflammatory effects through inhibition of oxidative burst in human monocytes", COLL ANTROPOL., 1 June 2012 (2012-06-01), pages 409 - 412, XP093081078, Retrieved from the Internet <URL:http://www.ncbi.nlm.nih.gov/pubmed/22856223> [retrieved on 20230912]
- YUSHKOV B G ET AL: "Changes in Erythropoiesis during Liver Regeneration after Partial Hepatectomy", BULLETIN OF EXPERIMENTAL BIOLOGY AND MEDICINE, KLUWER ACADEMIC PUBLISHERS-PLENUM PUBLISHERS, NE, vol. 139, no. 5, 1 May 2005 (2005-05-01), pages 554 - 557, XP019218056, ISSN: 1573-8221, DOI: 10.1007/S10517-005-0343-X

## Description

### BACKGROUND OF THE INVENTION

Some derivatives of Aminodihydrophthalazinedione sodium (ADPS), such as Tamerit^{™}, are used in Europe. Tamerit^{™} is an injectable human immunomodulator drug produced by Deko Co.

WO2017117586A1 relates to compositions that can be used, for example, in methods of treating medical conditions and symptoms associated with brain dysfunction, including but not limited to Gulf War illness (GWI), multiple chemical sensitivity (MCS), cognitive dysfunction (CD), multiple sclerosis (MS), and neurological disorders such as amyotrophic lateral sclerosis (ALS). The compositions comprise phthalazinediones or pharmaceutically acceptable salts thereof.

WO2017140422A1 relates to a method for producing a crystalline form of 5-amino-2,3-dihydro-1,4-phthalazinedione (luminol).

US2012329801A1 relates to the provision of two novel crystalline forms I and II for 5-amino-2,3-dihydrophthalazine-1,4-dione sodium salt.

US2002198208A1 relates to a medicinal preparation 5-amino-2,3-dihydrophthalazine-1,4-dione sodium salt having immunomodulatory, antiinflammatory, and antioxidant properties, and processes for manufacturing thereof.

Yushkov, B.G., Abidov, M.T., Danilova, I.G. et al. Changes in Erythropoiesis during Liver Regeneration after Partial Hepatectomy. Bull Exp Biol Med 139, 554-557 (2005) relates to changes in erythropoiesis during liver regeneration after partial hepatectomy depended on functional activity of phagocytosing mononuclear cells.

US2003195183A1 relates to immunocorrection of the organism using pharmacologically appropriate amino derivatives of 2,3-dihydrophthalazine-1,4-dione.

US8536171B2 relates to a method for obtaining 5-amino 2,3-dihydrophthalazine-1,4-dione alkali metal salts and their use in medicine.

US5543410A discloses the treatment of hepatitis B of viral origin by rectal administration of a suppository containing 100 mg of sodium salt of 5-aminophtaloylhydrazide.

### SUMMARY OF THE INVENTION

The present invention concerns an oral pharmaceutical composition, as defined in the appended claims, for use in the treatment of a disease in a subject in need thereof, the treatment comprising administering orally to a subject in need thereof of a therapeutically effective dose of the pharmaceutical composition, wherein the disease is non-viral hepatitis, drug-induced hepatitis or autoimmune hepatitis.

Aspects of the invention provide novel oral immediate release (IR) and extended release (ER) pharmaceutical compositions of aminodihydrophthalazinedione sodium (ADPS).

In some embodiments, the pharmaceutical composition can be used to enhance the immune system response. In some embodiments, the pharmaceutical composition has anti-inflammatory, antioxidant and/or immunomodulating properties.

The compositions provided herein have been made possible by the discovery according to some embodiments of this invention that ADPS exhibits significant intestinal and colonic adsorption.

In some embodiments, the pharmaceutical composition is an immediate release pharmaceutical composition. In other embodiments, the pharmaceutical composition is an extended release pharmaceutical composition.

Aspects of the invention relate to an oral composition comprising a therapeutically effective amount of aminodihydrophthalazinedione sodium (ADPS) and pharmaceutically acceptable excipients for use in the treatment of a disease in a subject in need thereof, the treatment comprising administering orally to a subject in need thereof of a therapeutically effective dose of the pharmaceutical composition, wherein the disease is non-viral hepatitis, drug-induced hepatitis or autoimmune hepatitis. In some embodiments, the composition for the use as claimed is formulated in a delivery mode selected from immediate release, extended release and combinations thereof. In some embodiments, the composition for the use as claimed comprises 10-1000 mg ADPS. In some embodiments, the composition for the use as claimed is formulated in a delivery mode selected from immediate release, extended release and combinations thereof. In some embodiments, the composition for the use as claimed comprises 50-1000 mg ADPS. In some embodiments, the composition for the use as claimed comprises 100-200 mg ADPS.

In some embodiments, the oral composition for the use as claimed comprising ADPS is prepared by granulation or by direct compression and is pH independent, while, the injection form has a pH in the range of 8-9.5.

In some embodiments, the composition for the use as claimed is prepared by direct compression process.

Aspects of the invention relate to an immediate release mode composition for the use as claimed. In some embodiments, the composition for the use as claimed comprises a core and a coating, wherein the core comprises 10-1000 mg ADPS, 100-500 mg of a filler, 5-50 mg of a binder, 5-80 mg of a disintegrant, 2-30 mg of a buffering agent, 5-20 mg of glidant, 3-10 mg of a lubricant and wherein the coating comprises 10-50 mg of a humidity protecting agent. In some embodiments, the composition for the use as claimed comprises a core and a coating, wherein the core comprises 100 mg ADPS, 100-250 mg of a filler, 5-20 mg binder, 5-30 mg of a disintegrant, 5-15 mg of a glidant, 3-7 mg of a lubricant and 2-20 mg of a buffering agent, and wherein the coating comprises 10-30 mg of a humidity protecting agent.

Aspects of the invention relates to an extended release mode composition for the use as claimed. In some embodiments, the composition for the use as claimed comprises a core and optionally a coating, wherein the core comprises 100-800 mg ADPS, 100-500 mg of a filler, 10-50 mg of a buffering agent, 50-500 mg of a matrix agent, 5-20 mg of a glidant and 3-10 mg lubricant and optionally 10-30 mg buffering agent and wherein the optional coating comprises 10-30 mg of a humidity protecting coating agent. In some embodiments, the composition for the use as claimed comprises a core and optionally a coating, wherein the core comprises 200 mg ADPS, 100-400 mg of a filler, 5-20 mg of a binder, 50-200 of a matrix agent, 5-30 mg baking powder, 5-15 mg of a glidant, 3-7 mg of a lubricant and optionally 10-30 mg buffering agent.

In some embodiments the extended release composition for the use as claimed is prepared by direct compression. In some embodiments the extended release composition for the use as claimed is prepared by wet granulation selected from low and high shear. In some embodiments the extended release composition for the use as claimed is in the form of tablet, minitablets, beads filled into capsules, beads compressed into tablets, or combinations thereof.

In some embodiments, the immediate release composition for the use as claimed or the extended release composition for the use as claimed comprises a basic buffering agent to maintain neutral pH and/or enable proper solubility of ADPS. In some embodiments, the buffering agent is sodium carbonate.

In some embodiments the filler is selected from mannitol, microcrystalline cellulose (Avicel 102), lactose for direct compression, dextrose, sorbitol, mannitol, maltitol, xylitol and any combination thereof.

In some embodiments, the binder is selected from povidone (PVP K25), maize starch, cellulose ethers (Methocell, Ethocel), Mg stearate, gelatin, sucrose and any combination thereof.

In some embodiments, the disintegrant is selected from PVP, dry potato starch, sodium starch glycolate, microcrystalline cellulose, magnesium stearate and any combination thereof.

In some embodiments, the glidant is selected from magnesium stearate, fumed silica, Aerosil 200, starch, talc and any combination thereof.

In some embodiments, the lubricant is selected from Mg stearate, talc, silica, fats, stearin, stearic acid and any combination thereof.

In some embodiments, the coating agent is selected from Sepifilm P 770, Opadry cosmetic coating and any combination thereof.

In some embodiments, the ADPS is in a form selected from anhydrous ADPS and ADPS dihydrate.

Aspects of the invention relate to the composition as claimed for use in a method of treatment of a disease in a subject in need thereof, by oral administration to the subject in need thereof of a therapeutically effective dose of the composition, wherein the disease is non-viral hepatitis, drug-induced hepatitis or autoimmune hepatitis. In some embodiments, the aminodihydrophthalazinedione sodium is administered in combination with a therapeutic agent. In some embodiments, the composition is for use in a method comprising administering a single dose of the pharmaceutical composition daily. In some embodiments, the single dose of 50-800 mg is administered daily for 5-50 days.

In some embodiments, the disease is non-viral hepatitis. In some embodiments, the disease is drug-induced hepatitis or autoimmune hepatitis.

### BRIEF DESCRIPTION OF THE FIGURES

Fig. 1 shows the dissolution profile of Tamerit IR formulation in two different media 0.1 M and pH 6.8 according to some embodiments.
Fig. 2 shows Tamerit dissolution results according to some embodiments
Fig. 3 shows porcine stomach Tamerit permeability results according to some embodiments
Fig. 4 shows Propranolol permeability stomach results according to some embodiments
Fig. 5 shows porcine small intestine permeability results according to some embodiments
Fig. 6 shows propranolol permeability small Intestine results according to some embodiments.
Fig. 7 shows porcine large intestine Tamerit permeability results according to some embodiments.
Fig. 8 shows Tamerit flux through GI membrane results according to some embodiments.
Fig. 9 shows propranolol flux through GI membrane results according to some embodiments.
Fig. 10 shows Tamerit flux vs Propranolol flux results according to some embodiments.
Fig. 11 shows Tamerit permeability vs Propranolol permeability results according to some embodiments.

### DETAILED DESCRIPTION OF THE INVENTION

Aspects of the invention relate to oral pharmaceutical compositions comprising a therapeutically effective amount of aminodihydrophthalazinedione sodium (ADPS) and a therapeutically acceptable excipient for use in the treatment of a disease as claimed. In some embodiments, the oral pharmaceutical composition can be an immediate release (IR), an extended release (ER) composition or combination thereof. In some embodiment, the oral pharmaceutical composition comprises minodihydrophthalazinedione sodium (ADPS).

As used herein, the term "pharmaceutically acceptable excipient" refers to an excipient that may be administered to a subject (e.g., a patient), together with the aminodihydrophthalazinedione sodium salt, and which does not destroy the pharmacological activity thereof and is nontoxic when administered in doses sufficient to deliver a therapeutic amount or an effective amount of the aminodihydrophthalazinedione sodium salt.

The compositions provided herein have been made possible by the discovery according to some embodiments of this invention that ADPS exhibits significant intestinal and colonic adsorption.

According to some aspects, the invention relates to methods of treatment comprising administering orally the pharmaceutical composition. Thus, methods according to some embodiments allow for the first time the active agent such as ADPS to be administered in a patient-friendly mode of administration and the clinical practitioner has for the first time the option to prescribe the active agent in either immediate release or extended release mode.

As used herein, the term "effective amount" refers to the amount of a compound that, alone or in combination with an amount of a therapeutic agent, when administered as an oral formulation or to an animal or human with a disease results in reduction in disease activity, as defined below in various embodiments.

US patent No. 8,536,171 discloses a process for the preparation of ADPS pharmaceutical substance.

Tamerit^{™} carries out its powerful therapeutic effect by regulating the immune system, by inhibiting hyperactivated cells while stimulating other cells. The destabilization of immune system, due to both hyperactivation and lack of activation, is a critical factor in many physiological conditions.

For a better understanding of the unique properties of Tamerit^{™} and its main differences from other drugs, it is necessary to understand therapeutic approaches to treat the immune system. Drugs affecting the immune system are divided into immunosuppressants, immunostimulators and immunomodulators or immunocorrectors. Immunosuppressants suppress immune system reactions, immunostimulators enhance immunity, and immunocorrectors inhibit an excessive immune response and, at the same time, potentiate low-intensity immune reactions.

To date, there are approximately 200 therapeutic agents that affect the immune system. Most drugs belong to immunostimulators or immunosuppressants. These preparations are of limited usefulness in medical practice.

Only 8 therapeutic preparations, including Tamerit^{™}, belong to the class of immunocorrectors. However, it should be emphasized that Tamerit^{™} differs from other immunocorrectors.

Firstly Tamerit^{™} is highly competitive immunocorrector due to its ability to directly influence the function of the macrophage - the "conductor of the immunological orchestra" and to normalize the immune response. In addition, Tamerit^{™} possesses a pronounced anti-inflammatory activity (TNF inhibitor) through the reduction of pro-inflammatory cytokines. Therefore, Tamerit^{™} may be used not only for the treatment of chronic infectious diseases, but also during acute inflammation and it has been experimentally and clinically proven that Tamerit^{™} is effective in the treatment of acute and chronic viral hepatitis and tuberculosis (M.T. Abidov, B.S. Nagoyev, A.P. Drozhzhin, M.V. Nelyubov, M.H. Kushkhova, A.P. Petko, S.V. Kakunin, A.H. Khamukov, A.F. Bashtanenko, 2000, Appendix 3 to the journal: Bulletin of Experimental Biology and Medicine, Targeted immunocorrection: problems, prospects, " The effectiveness of Tamerit in treatment of viral hepatitis", pp 87-88).

Secondly, Tamerit^{™} has proven immunocorrective, anti-inflammatory and antioxidant pharmacological properties. Other drugs usually produce only one of these effects.

Finally, Tamerit^{™}, unlike other ADPS derivatives, is rapidly excreted and has extremely low toxicity. Therefore, Tamerit^{™} may be suitable for pediatric therapy, as well.

The pharmacological effects of Tamerit^{™} are associated with its ability to regulate the function of monocytes and macrophages. In the case of acute inflammatory diseases, Tamerit^{™} has been shown to suppress reversibly for 10-12 hours the hyperactivity of macrophages and reduce synthesis of pro-inflammatory cytokines (tumor necrosis factor, interleukin-1, interleukin-6), nitro compounds and reactive oxygen anion radicals. At the same time, Tamerit^{™} enhances phagocytosis and the microbicidal system of neutrophilic granulocytes in the focus of inflammation. In acute inflammatory diseases, Tamerit^{™} reduces the level of pro-inflammatory cytokines in plasma and increases the metabolic activity of neutrophilic granulocytes. (M.T. Abidov, A.V. Karaulov, 2000, Appendix 3 to the journal: Bulletin of Experimental Biology and Medicine, Targeted immunocorrection: problems, prospects. "On the new details of the pathogenesis of toxic-septic conditions", pp.7-10).

The antioxidant activity of Tamerit^{™} is due to the inhibition of oxygen intake by hyperactive macrophages. In the case of inhibition of macrophage function, the preparation reactivates cytokine-producing and antigen-presenting functions of the above cells. Simultaneously, it restores normal the function of T- and B-lymphocytes. Due to its ability to regulate the production of nitro compounds and c-GMP synthetase, Tamerit^{™} corrects, through the release of nitro compounds, vascular tone and prevents vascular spasm (M.T. Abidov, A.P. Khokhlov, A.P. Drozhzhin, V.P. Fisenko, J.B. Panezheva, A.P. Petko, V.I. Egorov, M.H. Kushkhova, 2000. Appendix 3 to the journal: Bulletin of Experimental Biology and Medicine, Targeted immunocorrection: problems, prospects. "The study of the antioxidant activity of Tamerit ", pp. 24-25).

Tamerit^{™} also stimulates the restoration of damaged tissues and improves epithelization of ulcers of the mucous membrane (M.T. Abidov, 2000. Appendix 3 to the journal: Bulletin of Experimental Biology and Medicine, Targeted immunocorrection: problems, prospects. "Immunotropic activity of Tamerit", pp. 11-19).

There is an unmet need in the art for oral ADPS drugs, in both the immediate and extended release mode.

ADPS was developed in the form of as injectable drugs. The active agent of Tamerit^{™} is a derivate of aminodihydrophthalazinedione sodium (ADPS) and the dosage of the marketed product is 100 mg. The parenteral only mode of administration requires frequent doctor visits and unpleasant injections.

Aspects of the invention provide oral ADPS pharmaceutical compositions such as immediate release (IR) and/or extended release (ER) oral pharmaceutical compositions for the use as claimed. Oral compositions, unlike injectable ones, are easier to administer and lead to better patient compliance. In addition, the novel extended release form of administration of ADPS enables more convenient regimens of administration, like for example once daily administration regimen.

The novel oral compositions are based on the discovery of this invention that ADPS surprisingly exhibits colonic absorption, as will be demonstrated in this disclosure.

In addition, the inventors have found that ADPS has a very short half-life, which renders the presently used ADPS parenteral administration less effective.

The novel extended release compositions of aspects of the invention provide an effective solution to this problem.

### Characterization of the ADPS active agent

A number of commercially available ADPS samples were analyzed, characterized and compared (see Example 1, Tables 1-3). Analytical procedures for ADPS characterization were developed.

As a result of the characterization of the commercially available of chemical compounds, ADPS derivatives, it was concluded that the structures of Tamerit^{™} and other ADPS derivatives differs in content of water and TGA (thermogravimetric analysis), DSC (differential scanning calorimeter) peaks.

The compositions of aspects of this invention were developed using the commercially available active chemical component ADPS, which was identified as ADPS dihydrate (Tamerit^{™}).

### Development of the immediate release (IR) compositions

Two alternative technologies were tested:
a. direct compression
b. fluid bed granulation

In some embodiments, the process comprises of two steps:
a. the preparation of a core, and
b. coating the core with a humidity protecting agent.

Capsule shaped non-scored punches (19.5*9mm) were used. Hardness around 10 kg in average and NMT 15 minutes disintegration time.

After a few granulation and direct compression attempts, the direct compression technology was selected.

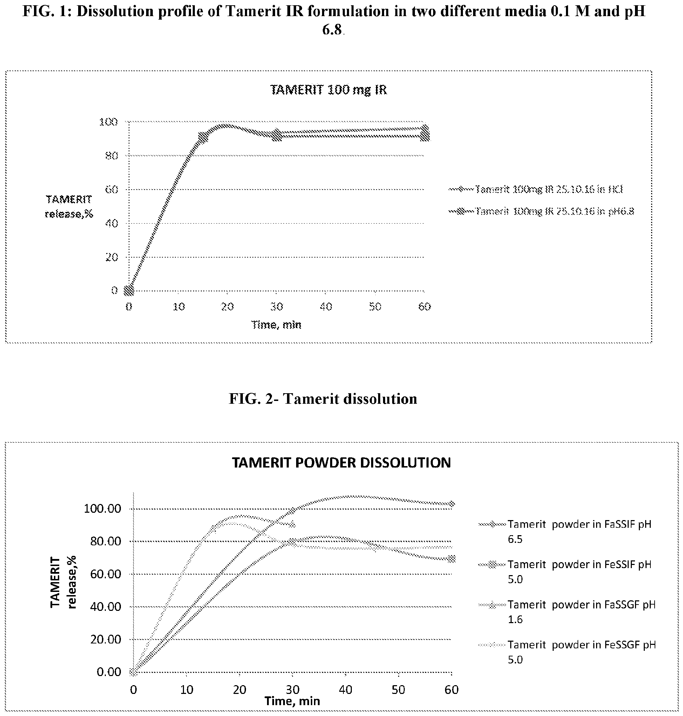

A pH independent, direct compression composition was developed (see Example 2, Fig. 1 and Table 4).

In some embodiments, the immediate release composition for use as claimed comprises a core and a coating, wherein the core comprises ADPS, a filler, a binder, a disintegrant, a glidant, a lubricant and optionally a buffering agent, and the coating comprises a humidity protecting coating agent.

In some embodiments, the immediate release composition for use of this invention comprises a core and a coating, wherein the core comprises 10-1000 mg ADPS, 100-500 mg of a filler, 5-50 mg binder, 5-80 mg disintegrant, 5-20 mg glidant, 3-10 mg lubricant and optionally 2-30 mg buffering agent, and the coating comprises 10-50 mg of a humidity protecting coating agent.

In some embodiments, the immediate release composition for use of this invention comprises a core and a coating, wherein the core comprises 50-1000 mg ADPS, 100-500 mg of a filler, 5-50 mg binder, 5-80 mg disintegrant, 5-20 mg glidant, 3-10 mg lubricant and optionally 2-30 mg buffering agent, and the coating comprises 10-50 mg of a humidity protecting coating agent.

In some embodiments, the immediate release composition for use of this invention comprises a core and a coating, wherein the core comprises 100-200 mg ADPS, 100-500 mg of a filler, 5-50 mg binder, 5-80 mg disintegrant, 5-20 mg glidant, 3-10 mg lubricant and optionally 2-30 mg buffering agent, and the coating comprises 10-50 mg of a humidity protecting coating agent.

In some embodiments, the immediate release composition for use of this invention comprises a core and a coating, wherein the core comprises 100 mg ADPS, 100-500 mg of a filler, 5-50 mg binder, 5-80 mg disintegrant, 5-20 mg glidant, 3-10 mg lubricant and optionally 2-30 mg buffering agent, and the coating comprises 10-50 mg of a humidity protecting coating agent.

In some embodiments, the immediate release composition for use of this invention comprises a core and a coating, wherein the core comprises 200 mg ADPS, 100-500 mg of a filler, 5-50 mg binder, 5-80 mg disintegrant, 5-20 mg glidant, 3-10 mg lubricant and optionally 2-30 mg buffering agent, and the coating comprises 10-50 mg of a humidity protecting coating agent.

In some embodiments, the coat is a moisture-protective coating which contributes to the stability of the tablet. In some embodiments, the pharmaceutical composition for use comprises from 10 mg to 50 mg of humidity protecting coating agent, for example, 10, 15, 20, 25, 30, 35, 40, 45, or 50 mg of humidity protecting coating agent.

In some embodiments, the immediate release composition for use of this invention comprises a core and a coating, wherein the core comprises 100 mg ADPS, 100-250 mg of a filler, 5-20 mg binder. 5-30 mg disintegrant, 5-15 mg glidant, 3-7 mg lubricant and optionally 2-20 mg buffering agent, and the coating comprises 10-30 mg of a humidity protecting coating agent.

In some embodiments, the immediate release composition for use of the invention comprises a core and a coating, wherein the core comprises 100-200 mg ADPS, 100-250 mg of a filler, 5-20 mg binder, 5-30 mg disintegrant, 5-15 mg glidant, 3-7 mg lubricant and optionally 2-20 mg buffering agent, and the coating comprises 10-30 mg of a humidity protecting coating agent. The filler can be selected from mannitol, microcrystalline cellulose (Avicel 102), lactose for direct compression, dextrose, sorbitol, mannitol, maltitol, xylitol and any combination thereof.

The binder can be selected from povidone (PVP K25), maize starch, cellulose ethers (Methocell, Ethocel), Mg stearate, gelatin, sucrose and any combination thereof.

The disintegrant can be selected from PVP, dry potato starch, sodium starch glycolate, microcrystalline cellulose, magnesium stearate and any combination thereof.

The glidant can be selected from magnesium stearate, fumed silica, Aerosil 200, starch, talc and any combination thereof.

The lubricant can be selected from Mg stearate, talc, silica, fats, stearin, stearic acid and any combination thereof

The coating agent can be selected from Sepifilm P 770, Opadry cosmetic coating and any combination thereof.

Optionally, a buffering agent like sodium carbonate may be added to the composition, in order to maintain neutral pH (pH around 7).

A method for the determination of the dissolution rate of the compositions has been developed.

The dissolution profile of the IR composition shows that more than 85% of the active agent ADPS is released after 15 minutes in 0.1 HCl and pH 6.8 (see Fig. 1).

### Development of the extended release (ER) compositions

In addition to the IR solid dosage formulation, an extended release or sustained release (ER) composition was developed as well, using direct compression technology (see Example 3).

Capsule shaped non-scored punches (19.5*9mm) were used. Hardness around 15-20 kg in average.

In some embodiments, the extended release composition for use as claimed comprises a core and optionally a coating, wherein the core comprises ADPS, a filler, a binder, a disintegrant, a glidant, a lubricant and optionally a buffering agent, and the optional coating comprises a humidity protecting coating agent. In some embodiments, the IR and/or ER compositions can be for its efficiency and equipotency. In some embodiments, the prolonged release composition was developed using selected humidity and granulation conditions, and selected degree of strengthening during the pressing process.

In some embodiments, the extended release composition for use as claimed comprises a core and optionally a coating, wherein the core comprises 100-800 mg ADPS, 100-500 mg of filler, 5-50 mg binder. 5-80 mg disintegrant, 50-500 of matrix agent, 3-10 mg lubricant, 5-20 mg glidant and optionally 10-50 mg buffering agent and a coating comprising 10-50 mg of a humidity protecting coating agent.

In some embodiments, the extended release composition for use of this invention comprises a core and a coating, wherein the core comprises 200 mg ADPS, 100-400 mg of filler, 5-20 mg binder, 50-200 of matrix agent, 5-30 mg disintegrant, 5-15 mg glidant, 3-7 mg lubricant and optionally 10-30 mg buffering agent and a coating comprising 10-30 mg of a humidity protecting coating agent.

The filler can be selected from mannitol, microcrystalline cellulose (Avicel 102), lactose for direct compression, dextrose, sorbitol, mannitol, maltitol, xylitol and any combinations thereof.

The binder can be selected from povidone (PVP K25), maize starch, cellulose ethers (Methocell, Ethocel), Mg stearate, gelatin, sucrose and any combinations thereof.

The disintegrant can be selected from PVP, dry potato starch, sodium starch glycolate, microcrystalline cellulose, magnesium stearate and any combinations thereof.

The glidant can be selected from magnesium stearate, fumed silica, Aerosil 200, starch, talc and any combinations thereof.

The lubricant can be selected from Mg stearate, talc, silica, fats, stearin, stearic acid and any combinations thereof.

The optional coating agent can be selected from Sepifilm P 770, Opadry cosmetic coating and any combinations thereof.

Optionally, a buffering agent like sodium carbonate may be added to the composition, in order to maintain neutral pH.

The coating of the ER composition is also optional.

Dissolution profiles were prepared in two different media 0.1 M and 6.8 pH and quite substantial differences are observed in dissolution profiles due to dependency of solubility of API.

### ADPS (Tamerit^{™}) Stability and Permeability Study

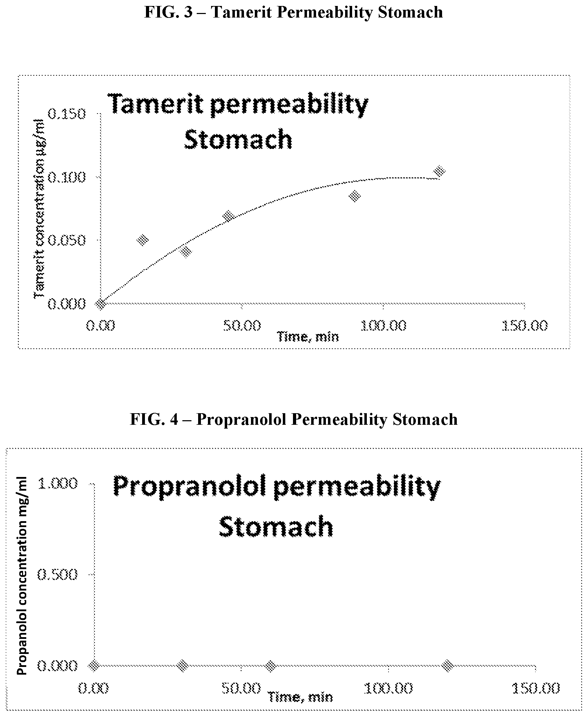

The stability and permeability of the active pharmaceutical agent ADPS (Tamerit^{™}) was evaluated in a porcine GI model, using Propranolol as a positive control (Example 4, Figs. 3-11, Tables 5-7).

### Objectives

- Evaluation of Tamerit's stability in Fasted and Fed states simulated gastric fluid (FaSSGF & FeSSGF);
- Evaluation of Tamerit's stability in Fasted and Fed state simulated intestinal fluid (FaSSIF & FaSSIF);
- Evaluation of Tamerit's stability in simulated colonic fluid (SCoF);
- Evaluation of Tamerit's permeability through porcine stomach (cardia), small intestine, and colon tissues using appropriate media and Franz cell apparatus.
- Comparing results of Propranolol positive control with Tamerit data in order to evaluate Tamerit's permeability.

### Porcine GI

Westerhout J et al. (Eur J Pharm Sci. 2014 Oct 15; 63:167-77) evaluated the applicability of porcine intestinal tissue as an alternative for human intestinal tissue for permeability studies, since porcine intestinal tissue is highly comparable to human intestinal tissue based on the omnivore characteristics of both humans and pigs especially for gastrointestinal processes (e.g. transit time, pH, and surface area).

### Propranolol as positive control

Propranolol is classified as a non-cardioselective sympatholytic beta blocker. It is rapidly and completely absorbed, with peak plasma levels achieved about 1-3 hours after ingestion.

Propranolol is classified by BSC as class 1 drug (high solubility, high permeability). Propranolol is predominantly absorbed by a passive diffusion mechanism (Pretorius E and Bouic P 2009). Having high partition coefficient and permeated by passive transcellular transport, propranolol was chosen as positive control substance for permeation experiments.

The results of the porcine GI study are detailed in Example 4, Figs. 3-11, Tables 5-7.

### Conclusions of the Tamerit vs Propranolol Permeability Study

- Both ADPS (Tamerit) and Propranolol demonstrated almost no permeation through gastric tissues
- ADPS has good permeability through small and large intestinal tissues
- As ADPS flux characteristic resembles Propranolol flux, these results may indicate that studied ADPS derivative has high permeability. Passive transcellular transport should be considered.
- The pharmaceutical composition ADPS presents good permeability through the tissue of the colon for a long period of time reaching steady state after four hours.
- After 4 hours ADPS is absorbed three time better than Propranolol in the small intestine and as good as Propranolol in large intestine.
- It can be assumed that absorption of ADPS by the oral route will be as good as absorption of propranolol.
- Tamerit is less soluble then propranolol.
- A solution containing 10 mM of Tamerit is a supersaturated solution.
- No visual changes were observed in Tamerit donor and receptor compartment during the absorption study.
- Change in color of receptor compartment was detected after 24 hours in Propranolol permeability study

Summing up, ADPS exhibits large intestine (colonic) absorption, making it a good candidate for the ER compositions.

These results provide the scientific basis for the development of the novel compositions of embodiments of this invention.

In some embodiments, there is provided an oral composition comprising a therapeutically effective amount of aminodihydrophthalazinedione sodium (ADPS) and pharmaceutically acceptable excipients for use in the treatment of a disease in a subject in need thereof, the treatment comprising administering orally to a subject in need thereof of a therapeutically effective dose of the pharmaceutical composition, wherein the disease is non-viral hepatitis, drug-induced hepatitis or autoimmune hepatitis.

In some embodiments, there is provided an oral composition comprising a therapeutically effective amount of aminodihydrophthalazinedione sodium (ADPS) for the use as claimed, wherein formulated in a delivery mode selected from immediate release, extended release and combinations thereof.

In some embodiments, the composition comprises 10-1000 mg ADPS. In some embodiments, the composition comprises 50-1000 mg ADPS. In some embodiments, the composition comprises 50-500 mg ADPS. In some embodiments, the composition comprises 50-400 mg ADPS. In some embodiments, the composition comprises 50-300 mg ADPS. In some embodiments, the composition comprises 50-200 mg ADPS. In some embodiments, the composition comprises 100-500 mg ADPS. In some embodiments, the composition comprises 100-400 mg ADPS. In some embodiments, the composition comprises 100-300 mg ADPS. In some embodiments, the composition comprises 100-200 mg ADPS. In some embodiments, the composition comprises 10-500 mg ADPS. In some embodiments, the composition comprises 10-400 mg ADPS. In some embodiments, the composition comprises 10-300 mg ADPS. In some embodiments, the composition comprises 10-200 mg ADPS. In some embodiments, the composition comprises about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg ADPS. In some embodiments, the composition comprises from about 100 mg to about 200 mg ADPS. In some embodiments, the composition comprises from about 110 mg to about 200 mg ADPS. In some embodiments, the composition comprises from about 120 mg to about 200 mg ADPS. In some embodiments, the composition comprises from about 130 mg to about 200 mg ADPS. In some embodiments, the composition comprises from about 140 mg to about 200 mg ADPS. In some embodiments, the composition comprises from about 150 mg to about 200 mg ADPS. In some embodiments, the composition comprises from about 160 mg to about 200 mg ADPS. In some embodiments, the composition comprises from about 170 mg to about 200 mg ADPS. In some embodiments, the composition comprises from about 180 mg to about 200 mg ADPS. In some embodiments, the composition comprises from about 190 mg to about 200 mg ADPS. In some embodiments, the composition comprises from about 100 mg to about 190 mg ADPS. In some embodiments, the composition comprises from about 100 mg to about 180 mg ADPS. In some embodiments, the composition comprises from about 100 mg to about 170 mg ADPS. In some embodiments, the composition comprises from about 100 mg to about 160 mg ADPS. In some embodiments, the composition comprises from about 100 mg to about 150 mg ADPS. In some embodiments, the composition comprises from about 100 mg to about 140 mg ADPS. In some embodiments, the composition comprises from about 100 mg to about 130 mg ADPS. In some embodiments, the composition comprises from about 100 mg to about 120 mg ADPS. In some embodiments, the composition comprises from about 100 mg to about 110 mg ADPS.

In some embodiments, there is provided a composition for use as claimed, wherein the composition comprises 100-200 mg ADPS.

In some embodiments, the composition is prepared by granulation or direct compression process. In some embodiments, the process is pH independent.

In some embodiments, the composition is prepared by direct compression process.

In some embodiments, a composition is provided, wherein in immediate release mode and wherein said composition comprises a core and a coating, wherein the core comprises 50-1000 mg ADPS, 100-500 mg of filler, 5-50 mg binder, 5-80 mg disintegrant, 2-30 mg buffer agent, 5-20 mg glidant, 3-10 mg lubricant and the coating comprises 10-50 mg of a humidity protecting coating agent.

In some embodiments, a composition for use as claimed is provided, wherein in immediate release mode and wherein said composition comprises a core and a coating, wherein the core comprises 100 mg ADPS, 100-250 mg of filler, 5-20 mg binder, 5-30 mg disintegrant, 5-15 mg glidant, 3-7 mg lubricant and optionally 2-20 mg buffering agent, and the coating comprises 10-30 mg of a humidity protecting coating agent.

In some embodiments, a composition for use as claimed is provided, wherein in extended release mode and wherein comprising a core and optionally a coating, wherein the core comprises 100-800 mg ADPS, 100-500 mg of filler, 10-50 mg buffer agent, 50-500 mg of matrix agent, 5-20 mg glidant and 3-10 mg lubricant and optionally 10-30 mg buffering agent and a coating comprising 10-30 mg of a humidity protecting coating agent.

In some embodiments, a composition for use as claimed is provided, wherein in extended release mode and wherein comprising a core and optionally a coating, wherein the core comprises 200 mg ADPS, 100-400 mg of filler, 5-20 mg binder, 50-200 of matrix agent, 5-30 mg disintegrant, 5-15 mg glidant, 3-7 mg lubricant and optionally 10-30 mg buffering agent and a coating comprising 10-30 mg of a humidity protecting coating agent.

In some embodiments, there is provided an extended release composition for use as claimed, wherein the composition is prepared by direct compression.

In some embodiments, there is provided an extended release composition for use as claimed wherein the composition is prepared by wet granulation selected from low and high shear.

In some embodiments, there is provided an extended release composition for use as claimed, wherein the composition is in the form of tablet, minitablets, beads filled into capsules, beads compressed into tablets, or combinations thereof.

In some embodiments, there is provided a composition for use as claimed wherein the composition further comprises a basic buffering agent to maintain neutral pH and enable proper solubility of ADPS.

In some embodiments, there is provided a composition for use as claimed, wherein the basic buffering agent is sodium carbonate.

In some embodiments, there is provided a composition for use as claimed, wherein the filler is selected from mannitol, microcrystalline cellulose (Avicel 102), lactose for direct compression, dextrose, sorbitol, mannitol, maltitol, xylitol and any combination thereof.

In some embodiments, there is provided a composition for use as claimed, wherein the binder is selected from povidone (PVP K25), maize starch, cellulose ethers (Methocell, Ethocel), Mg stearate, gelatin, sucrose and any combination thereof.

In some embodiments, there is provided a composition for use as claimed, wherein the disintegrant is selected from PVP, dry potato starch, sodium starch glycolate, microcrystalline cellulose, magnesium stearate and any combination thereof.

In some embodiments, there is provided a composition for use as claimed, wherein the glidant is selected from magnesium stearate, fumed silica, Aerosil 200, starch, talc and any combination thereof.

In some embodiments, there is provided a composition for use as claimed, wherein the lubricant is selected from Mg stearate, talc, silica, fats, stearin, stearic acid and any combination thereof.

In some embodiments, there is provided a composition for use as claimed, wherein the coating agent is selected from Sepifilm P 770, Opadry cosmetic coating and any combination thereof.

In some embodiments, there is provided the active agent ADPS of this invention, wherein in a form selected from anhydrous ADPS and ADPS dihydrate.

In some embodiments, there is provided a composition as claimed for use in a method of treatment of a disease in a subject in need thereof, by oral administration to a subject in need thereof of a therapeutically effective dose of a composition of any aspects of this invention, wherein the disease is non-viral hepatitis, drug-induced hepatitis or autoimmune hepatitis.

An effective oral dose may be given daily (in one or divided doses), three times weekly, two times weekly, or monthly to a subject in need thereof, human or non-human.

### Gastroenterology application

In some embodiments, the composition provided herein can be used to stimulate the regeneration of hepatocytes in various injuries of the liver. For example, the composition can be used in gastroenterology for the treatment of acute and chronic non-viral hepatitis.

The deterioration of the ecological situation, failure of the population to maintain a healthy lifestyle, numerous bad health habits (smoking, alcoholism, substance abuse, drug addiction), contribute to the wide spread of various forms of hepatitis.

The frequency of prevalence of acute, non-viral, chronic hepatitis, the lack of effective drug therapy, the difficulty in predicting the clinical course, the adverse effects of the disease with disability, incapacitation and mortality determine the search for new approaches to the treatment of this pathology. A method may be promising, based on producing effect on hepatocytes in order to preserve and restore their functional and proliferative abilities.

Damage to the structure of the liver and pancreas and other organs resulting from the use of poor-quality alcohol, alcohol-containing liquids, drugs, industrial poisons, pesticides, food preservatives is characterized by the activation of free-radical oxidation, destruction of enzyme systems, deep metabolic restructuring of oxidative processes, which leads to heavy, functional and morphological, sometimes irreversible changes in the organs against the background of the formed autoimmune process.

Currently, the search for effective methods of correcting the above conditions is carried out in the direction of a possible influence through the system of cell-cell interactions on the regulation of metabolism and the regeneration of hepatocytes. There are a significant number of drug treatments for this kind of hepatitis. Depending on the mechanism of action, hepatoprotectors are divided into 4 classes:
1. antioxidants 2. xenobiotic metabolism inhibitors 3. phospholipid preparations that regenerate hepatocyte membranes 4. stimulators of metabolic processes etc.

In the treatment of acute hepatitis, diet and treatment with coenzyme, metabolic and multivitamin preparations are indicated. Heptral (adenosyl-alpha-methionine) is widely used, which reduces the degree of mitochondrial damage and promotes the restoration of glutathione. A course of intravenous injections of Essentiale 5-10 ml in 5% glucose solution - 10-30 injections can be prescribed. At the same time Essentiale is ingested 2 capsules 3 times a day for 1-2 months and then 3-4 capsules a day for up to 6 months. Membrane stabilizing drugs are also indicated: Legalon and Catergen for 1-2 months. In addition, detoxification therapy is indicated - intravenous infusion of 5% glucose solution, Hemodez, Ringer's solution. The use of biotechnological probiotic products in combination with antioxidants (vitamin C, B, E) is considered promising.

A disadvantage of the known methods of treatment of acute and chronic hepatitis is their low efficacy due to the need to use a large range of drugs with a multidirectional mechanism of action over a long period of time, which in turn causes an additional xenobiotic load on the liver and organs of excretion and neutralization, resulting in disorder of life support systems and a large number of serious complications.

There is also a method of treating chronic hepatitis and cirrhosis by cannulating the umbilical vein and endolymphatic administration of drugs with the additional administration of the dispersed biomaterial.

This method is carried out as one of the stages of the operation and is more complex and time-consuming manipulation and is not widely tested.

The closest is a method of treating acute toxic hepatitis by prescribing comprehensive treatment - Duphalac at a dose of 30-50 ml 3 times a day, Hepasol A - 1000 ml per day, Heptral - 800 mg per day by intravenous infusion, Prednisolone - 240 mg per day, Metadoxil - 600-900 mg per day by intravenous infusion and detoxification infusion therapy (infusion of Ringer's solution).

Open and closed liver damage are severe injuries. Among the causes of liver damage in peacetime, transport and household injuries occupy a leading place (72-87%), industrial injuries account for 13-22%. Tactics of treatment in all cases of injury requires surgery, the outcome of which depends on the nature of the injury. High compensatory abilities of the liver ensure restoration of its function even after extensive damage. The manifestation of reparative processes can be detected only 10-15 days after injury, and the final normalization of the liver structure occurs 30-180 days later. Treatment of liver failure starts with the restoration of the function of hepatocytes and improvement of hepatic blood flow. Hepatoprotectors (Essentiale, Heptral) and multivitamins are prescribed.

In some embodiments, the technical problem can be, at the first stage, the inhibition of hyperactivity of hepatic macrophages with a subsequent decrease in the generation of reactive oxygen anion radicals and, at the next stage, the stimulation of the regeneration of hepatocytes through influencing the macrophages by one of the derivatives of 5-aminophthalhydrazide, Tamerit, in the experiment and in the clinic with the general formula:
In some embodiments, the composition stimulates the regeneration of hepatocytes, and in the case of combination therapy there is a synergistic effect from the interaction of Tamerit with any drugs traditionally used in the treatment
of hepatitis, which leads to a shortening of the treatment of hepatitis of different nature and full restoration of liver functions.

The technical result can be achieved due to the additional administration, against the background of basic therapy, of macrophage function regulator, TNF inhibitor Tamerit, daily, at a dose of 10-800 mg per day, for 15-50 days, depending on the severity and intensity of the pathological process in the form of enteric coated tablets or as injections, depending on the severity of the patient.

At the first stage of development of the pathological process, toxicants, antigens are the trigger mechanism, and then the outcome of the pathological process depends on the response of the body, i.e. the main effector cell - hepatic macrophage. As to the treatment of non-viral, exotoxic hepatitis, it was shown that to protect the liver from various damaging factors, the best choice is to include in the treatment regimen the drug Tamerit (as an analogy of non-steroidal anti-inflammatory agents), which actively reduces the level of pro-inflammatory cytokines responsible for the development of symptoms of intoxication, toxic and septic condition, shock.

The essence of aspects of the disclosure lies in the fact that the proposed pharmaceutical composition for use in method for the treatment of acute, chronic, post-viral, autoimmune hepatitis (hepatopancreatitis) and other liver damage through stimulation of hepatocyte regeneration does not have adequate analogues, since it aims to regulate the activity of the liver cells, which causes the restoration of the structural and functional state of the liver with minimal drug load on the body.

In this case, the tactics of treating liver diseases of different etiological factors suggest that along with a specific basic therapy (antidotes), the patient is treated with Tamerit in the form of enteric coated tablets at a dose of 10-800 mg per day. The course of the drug administered depends on the severity of the disease and is at least 5-150 doses. In some embodiments, Tamerit can be used as a monotherapy in the same way as in combination therapy. The drug can be administered in the form of tablets in various doses. In some embodiments, Tamerit can be used in combination with any other auxiliary drugs.

The pharmaceutical composition for use in methods of treatment disclosed herein reduce the inflammatory response in the liver tissue, which develops with the above-mentioned diseases up to its complete relief, help to reduce the symptoms of exogenous and endogenous intoxication, and also cause the regeneration of hepatocytes, increasing their functional activity.

Macrophages are cells of the immune response and markers of inflammation. It was shown that in the early stages of damage, activation of macrophages accompanies alteration and inflammation processes due to the secretion of a large number of pro-inflammatory and cytotoxic mediators, such as TNF-α, interleukins 1, 2, 6, nitric oxide, reactive oxygen species, eicosanoids, and so on. It is these mediators and cytokines secreted by activated macrophages that induce apoptosis and necrosis of hepatocytes. On the other hand, fragments of destroyed cells and components of their cytosol are autoantigens, which in turn enhance inflammation and the immune response. Further formation of the pathological process can be traced as follows; antibodies are generated against autoantigens and a circulating immune complex (CIC) is formed in the presence of complement. Further, the CIC may exit the focus of inflammation and contribute to the formation of a new focus in other organs. If the CIC remain in the liver, they attract "alien" macrophages and leukocytes from the blood stream, which contribute their destructive potential to the pathological process, thereby contributing to the formation of a vicious circle. In case of intoxication with toxicants, the process may follow a different scenario. Toxicants cause activation of phospholipases with disruption of membrane permeability, cell destruction, release of hydrolases, a sharp increase in lysosomal enzymes, a change in pH, an increase in electric potential of the cell in the focus of inflammation. All this as a whole contributes to the development of hypoxia, activation of lipid peroxidation (LPO), damage to the cell, disseminated intravascular coagulation syndrome (DIC syndrome), and massive death of liver cells. Moreover, in the case of "alien" macrophage involvement in the pathological process, the situation gets out of control, the process acquires cascade irreversible character. Hepatic, renal and multiple organ failure develop with all the ensuing consequences. Progressive inflammation is a serious problem in the pathogenesis of liver diseases, and then diseases of the pancreas, because it always promotes development of fibrosis, cirrhosis of the liver and is one of the main causes of hepatocellular carcinomas.

The 5-aminophthalhydrazide derivative, Tamerit, by inhibiting for a short time the synthesis of pro-inflammatory cytokines, reactive radicals, leads to the breaking of the pathogenetic chain, reduces inflammation, and stimulates the regeneration in the organ tissue (M.T. Abidov, B.S. Nagoyev, A.P. Drozhzhin, M.V. Nelyubov, M.H. Kushkhova, A.P. Petko, S.V. Kakunin, A.H. Khamukov, A.F. Bashtanenko, 2000, Appendix 3 to the journal: Bulletin of Experimental Biology and Medicine, Targeted immunocorrection: problems, prospects, »The effectiveness of Tamerit in treatment of viral hepatitis« , pp 87-88, M.T.Abidov, Professor B.S. Nagoyev, Zhanna Betovna Ponezheva, «Efficacy of Tamerit for clinical and immunological parameters of patients with chronic hepatitis C», 2011, p. 115-141).

In addition, macrophages, performing anti-infective protection, support the liver tissue homeostasis and stimulate regeneration processes in it.

Aspects of the disclosure allow for effective treatment of acute chronic hepatitis (hepatopancreatitis) and to overcome the risk of possible complications of these diseases.

Some aspects relate to uses in methods of treating hepatitis with aminophthalhydrazide derivative either as monotherapy or as part of combination therapy.

Aspects of the invention relate to a pharmaceutical composition for use in a method of treatment of a disease in a subject in need thereof, by oral administration to the subject in need thereof of a therapeutically effective dose of the composition, wherein the disease is non-viral hepatitis, drug-induced hepatitis or autoimmune hepatitis. In some embodiments, the aminodihydrophthalazinedione sodium is administered in combination with a therapeutic agent. In some embodiments, the pharmaceutical composition is for use in a method comprising administering a single dose of the pharmaceutical composition daily. In some embodiments, the single dose of 50-800 mg is administered daily for 5-50 days.

### EXAMPLES

The following examples illustrate certain embodiments of the invention but are not meant to limit the scope of the claims in any way. The following examples are put forth so as to provide those of ordinary skill in the art with a complete disclosure and description of how to make and use the described invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### EXAMPLE 1

Characterization of some analogs of pharmaceutical raw materials ADPS and chemicals Four suppliers of commercial ADPS materials were found:

### Suppliers:

| **Supplier** | **Quantity** |
|---|---|
| Tamerit | 500 g |
| Luminol Sodium Salt Sigma (#884) | Small sample |
| Luminol Sodium Salt Chemleader (#97) | Small sample |
| Luminol Sodium Salt WuXi AppTec (#98) | Small sample |

Based on the HPLC evaluation of area under the peaks, the active agents are compared in **Table 1.**

**Table 1: Comparison between results of ADHC HPLC evaluation**

| **Stock no.** | **Tamerit** | **Luminol Sodium Salt Sigma (#884)** | **Luminol Sodium Salt Chemleader (#97)** | **Luminol Sodium Salt WuXi AppTec (#98)** |
|---|---|---|---|---|
| **Solution Prep. Date** | 17.08.16 | 18.08.16 | 18.08.16 | 18.08.16 |
| **Standard Weight, mg** | 10.85 | 10.10 | 11.20 | 10.60 |
| **Volume,** ml | 100 | 100 | 100 | 100 |
| **Purity, %** | 100 | 99 | 98 | 98 |
| **TAM ST conc., mg/ml** | **0.1085** | **0.1000** | **0.1098** | **0.1039** |
| **Peak Area inj-1** | **48.7480** | **49.1810** | **46.2420** | **41.2710** |
| **Peak Area inj-2** | **47.9730** | **49.2570** | **46.2140** | **41.3200** |
| **AVG** | **48.361** | **49.219** | **46.228** | **41.296** |
| **%Difference** | **0.00** | **10.44** | **-5.51** | **-10.81** |

### DSC - Differential scanning calorimetry

### Tamerit

The thermal behavior by DSC and water contents by Karl Fisher of material received and the following results were obtained for Tamerit:
Water content is 11.7%,
DSC pattern (Figure 1) showed two events:
   peak 1 at 82°C which can be explained to absorbed water;
   peak 2 at 94 °C which can be explained to bound water (hydrates)

Based on the results, it can be stated that Tamerit (sodium salt of aminophthalhydrazide) differs in the studied physical and chemical properties from luminol sodium salts which are classified as chemical reagents.

The same DSC method was used to determine all other samples.

### TGA - Thermogravimetric analysis

Thermogravimetric analysis (TGA) is a method in which changes in physical and chemical properties of materials are measured as a function of increasing temperature (with constant heating rate), or as a function of time (with constant temperature and/or constant mass loss).

Based on the results of measured samples the following thermogravimetric results were obtained:

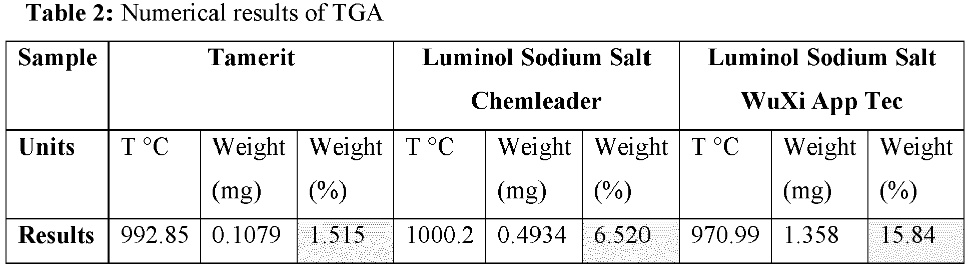

In addition to TGA and DSC, determination of water was checked using Karl Fischer method. The results shifted from 0.8 to 14% in water therefore it can be stated that the Sigma sample is not a pharmaceutical product such as Tamerit.

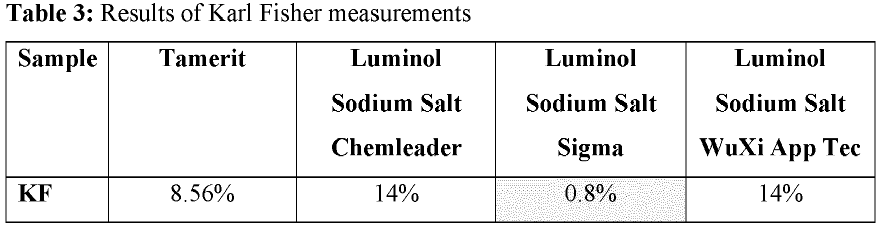

### Conclusions on ADPS identity:

Pharmaceutical product and chemistries differ in water content and peaks of TGA and DSC.

### EXAMPLE 2- IMMEDIATE RELEASE (IR) ORAL COMPOSITION

Two technologies were tested:
a. direct compression
b. fluid bed granulation

After few granulation and direct compression attempts the following pH independent formulation was developed, based on direct compression technique, at this stage uncoated.

Capsule shaped non-scored punches (19.5*9mm) were used. Hardness around 10 kg in average and NMT 15 minutes disintegration time.

### IR compositions

1. The immediate release composition comprises a core and a coating, wherein the core comprises ADPS, a filler, a binder, a disintegrant, a glidant, a lubricant and optionally a buffering agent, and the coating comprises a humidity protecting coating agent.
2. The immediate release composition of aspects of the invention comprises a core and a coating, wherein the core comprises 10-1000 mg ADPS, 100-500 mg of filler, 5-50 mg binder, 5-80 mg disintegrant, 5-20 mg glidant, 3-10 mg lubricant and optionally 2-30 mg buffering agent, and the coating comprises 10-50 mg of a humidity protecting coating agent.
3. The immediate release composition of aspects of the invention comprises a core and a coating, wherein the core comprises 100 mg ADPS, 100-250 mg of filler, 5-20 mg binder, 5-30 mg disintegrant, 5-15 mg glidant, 3-7 mg lubricant and optionally 2-20 mg buffering agent, and the coating comprises 10-30 mg of a humidity protecting coating agent.

Optionally, a buffering agent like sodium carbonate may be added to the composition, in order to maintain neutral pH.

A method for the determination of the dissolution rate of the compositions has been developed.

Three dissolution points were checked 15 min, 30 min and 60 min with following results:

**Table 4 - Dissolution of IR composition**

| **Tamerit 100 mg IR** | |
|---|---|
| Tamerit 15 min | 86 |
| Tamerit 30 min | 90 |
| Tamerit 60 min | 92 |

More than 85% release of active substance is achieved after 15 minutes in 0.1 HCl and pH 6.8 (see FIG. 1).

### EXAMPLE 3 - EXTENDED RELEASE (ER) ORAL COMPOSITION

In addition to IR solid dosage formulation an extended release (ER) formulation was prepared using direct compression technology, using the below composition.

Capsule shaped non-scored punches (19.5*9mm) were used. Hardness around 15-20 kg in average.

Samples were also placed at 3 months the stability study in the following research conditions:
- 25°C ± 2 °C / 60 % RH ± 5% RH 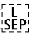 - 40°C ± 2 °C / 75 % RH ± 5% RH

### ER compositions

1. In some embodiments, the extended release composition comprises a core and optionally a coating, wherein the core comprises ADPS, a filler, a binder, a disintegrant, a glidant, a lubricant and optionally a buffering agent, and the optional coating comprises a humidity protecting coating agent.
2. In some embodiments, the extended release composition comprises a core and optionally a coating, wherein the core comprises 10-800 mg ADPS, 100-500 mg of filler, 5-50 mg binder, 5-80 mg disintegrant, 50-500 mg of matrix agent, 3-10 mg lubricant, 5-20 mg glidant and optionally 10-50 mg buffering agent and a coating comprising 10-50 mg of a humidity protecting coating agent.
3. In some embodiments, the extended release composition comprises a core and a coating, wherein the core comprises 200 mg ADPS, 100-400 mg of filler, 5-20 mg binder, 50-200 mg of matrix agent, 5-30 mg disintegrant, 5-15 mg glidant, 3-7 mg lubricant and optionally 10-30 mg buffering agent and a coating comprising 10-30 mg of a humidity protecting coating agent.

Optionally, a buffering agent like sodium carbonate may be added to the composition, in order to maintain neutral pH. In some embodiments, the coat may be added to the ER composition.

Dissolution profiles were prepared in two different media 0.1 M and 6.8 pH and quite substantial differences were observed in dissolution profiles due to dependency of solubility of API.

### EXAMPLE 4

### ADPS Stability and Permeability in Porcine Models

### Study Objectives

- Evaluation of ADPS stability in Fasted and Fed states simulated gastric fluid (FaSSGF & FeSSGF);
- Evaluation of ADPS stability in Fasted and Fed state simulating intestinal fluid (FaSSIF & FaSSIF);
- Evaluation of ADPS stability in simulated colonic fluid (SCoF);
- Evaluation of ADPS permeability through porcine stomach (cardia), small intestine, and column tissues using appropriate media and Franz cell apparatus Comparison of results of Propranolol positive control results with ADPS data to extrapolate the ADPS absorbability.

### Materials and Methods

### Materials

### Receiver Compartment Buffer

### Name: Dulbecco's Phosphate Buffered Saline

| | |
|---|---|
| Manufacturer: | Biological industries |
| Catalog No.: | 02-020-1A |
| Physical State: | Liquid |
| Supplied by: | Biological industries |
| Storage Conditions: | 2-8°C |
| Name in the report: | Phosphate Buffer |

### Donor Compartment Buffers and Dissolution Buffers

### Title: FaSSGF: Fasted state simulating gastric fluid

### Preparation FaSSGF (for 2000ml)

Pepsin 500mg (Item QC-15, Pepsin porcine gastric mucosa, powder, ≥250 units/mg solid, Sigma P7000)
4gr NaCl (Item QC-5, Sigma 746398)
FAS powder 0.12g (Biorelevant FFF02)
Adjust pH to 1.6 with HCL (1M)
Name: FeSSGF: Fed state simulating gastric fluid
NaCl 237.02mM
Acetic acid 17.12mM
Sodium acetate 29.75mM
1:1 with milk (v/v)
pH 5 with NaCl/Hydrochloric acid
Name: FaSSIF Fasted state simulating intestinal fluid
   Preparation of 100ml solution
   FaSSCoF 25mg - Biorelevant lot 01-1701A-02NP #QC-14
   NaH₂PO*2H₂O 0.45g Sigma 04269 lot #SZBF0150V #833
   NaOH 0.04g Merck 106469 lot B0999069 #811
   NaCl 0.62g Sigma 746398 lot SLBP3513V
   Adjust pH to 6.5 with HCL (1M)
   Pancreatin 22.5mg Sigma P1625 lot SLBK9748V #825
Name: FeSSIF Fed state simulating intestinal fluid
For preparation of 2L media
Prepared 1L buffer
NaCl 237.02mM (13.9g)
Acetic acid 17.12mM ( ]1.03g)
Sodium acetate 29.75mM (2.44g)
1:1 with milk (v/v)
pH adjusted to pH 5 with NaCl/Hydrochloric acid
Name: SCoF (Simulated colonic fluid)
Preparation of 100ml solution
   Buffer:
   Sodium Hydroxide 0.066g
Maleic Acid 0.35g
Tris base 0.369g
Adjust pH with HCl 1N or NaOH 1N to **pH 6.0**
FeSSCoF 0.074g
Water to 100ml
(Should stand 1h prior to use)
Prior to testing
Lactobacilli drops (100 million per dissolution /20million per permeation donor compartment) added

### Test Items (Permeation study)

Tamerit in donor compartment 10mM (2.3mg/ml)
Propranolol in donor compartment 10mM (2.6mg/ml)
   Flow rate: 1.2 ml/min; Temperature: 40°C;
   Injection volume: 5 µl; UV detector: λ = 220 nm; 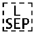 Diluent: water 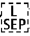 RT [retention time] of Tamerit: about 2.8 min
   Preparations:
      Standard solution: about 0.02 mg/mL of Tamerit in water.
      Sample solution: 2.0 ml of sampling solution dilute to 10.0 ml with water.
      HPLC Assay of Propranolol

### Instrument conditions

Column: Phenomenex, Gemini^{®} 5 µm C18 110A, 250×4.6 mm
Mobile phase:
   ACN:MeOH: 0.01M disodium hydrogen phosphate dihydrate (pH 3.5) (50:35:15 v/v) Flow rate: 0.5 mL/min

**Table 5 - Solvents Gradient Table:**

| Time, min | MP A, % | MP B, % |
|---|---|---|
| 0 | 100 | 0 |
| 0.4 | 100 | 0 |
| 3.4 | 20 | 80 |
| 3.85 | 0 | 100 |
| 3.86 | 100 | 0 |
| 8.0 | 100 | 0 |

Temperature: 25°C;
Injection volume: 20 µL;
UV detector: λ=290 nm;
Diluent: mobile phase
RT of PROPRANOLOL: about 5.1 min

### Preparations:

**Standard solution:** about 0.01 mg/mL of propranolol in diluent.
**sample solution:** about 0.01 mg/ml of propranolol in diluent

### Equipment

- Dissolution tester Varian 705DS equipped with Distec Syringe Pump and Evolution 4300 Dissolution Sampler. Apparatus 2- Paddle, SB-A-058
- HPLC system Dionex Ultimate 3000, SB-A-002
- Analytical balance, RADWAG, SB-A-059
- Magnetic Stirrer, Fried electric, SB-P -026/4
- 20 µ high molecular weight polyethylene filters incorporated into dissolution cannula (on-line filter). Dissolution accessories, Cat. Nº PSFIL020-01
- Nylon 0.45µ Syringe filters, Membrane-solutions, Cat. #SFNY025045N or equivalent
- The glassware used was volumetric of class A quality.
- PermeGear Franz Cells with 9mm jacketed cells flat ground (ground o-ring) with 5ml receptor volume (cells catalog number 4G-01-00-09-05) contact surface of 0.64 square cm
- PermeGear V6B Stirrer
- Water pump: Freed Electric TEP-4

**Table 6 - Summary of GI Stability experiment procedures**

| **Tasks** | **Volume** | **Conditions** | **Time** |
|---|---|---|---|
| Evaluation of Tamerit stability in Fasted state simulating gastric fluid (FaSSGF). | 500ml, apparatus II 37°C | 500mL FaSSGF (from biorelevant) pH 1.6 + Pepsin 500mg | 15min |
| | | | 30min |
| | In triplicate 75 RPM | | |
| | | 100mg of Tamerit | |
| | Tamerit amount is 100 mg in each unit | (preparation of 2L of media: 4g of NaCl- > 1.8Lwater, adjust to pH1.6 with HCl(1N) Add 0.12g of FAS powder, dissolve and make up to volume 2L at RT) | |
| Evaluation of Tamerit stability in Fed state simulated gastric fluid (FeSSGF). | 500ml apparatus II 37°C | preparation of 2L of media | 15min |
| | | | 30min |
| | | preparation of 1L of buffer | 45min |
| | In triplicate 75 RPM | NaCl 237.02mM (13.9g) | 90min |
| | | Acetic acid 17.12mM (1.03g) | |
| | Tamerit amount is 100 mg in each unit | Sodium acetate 29.75mM (2.44g) | |
| | | 1:1 with milk (v/v) | |
| | | pH 5 with NaCl/Hydrochloric acid | |
| Evaluation of Tamerit stability in Fasted state simulating intestinal fluid (FaSSIF). | 500ml apparatus II 37°C | 500mL FaSSIF pH 6.5 + Pancreatin 450mg | 30min |
| | | | 60min |
| | | | 90min |
| | In triplicate 75 RPM | 100mg of Tamerit | 120min |
| | | | 180min |
| | Tamerit amount is 100 mg in each unit | (preparation of 2L of media: 0.84g of NaOH pellets, 8.94g NaH2PO4x2H2O, 12.37g NaCl 1.8Lwater, adjust pH to 6.5 with or HCl(1N) or NaOH (1N) and make up to volume 2L | |
| | | Take 4.48g of FSIF powder, dissolve and make up to volume 2L at RT). | |
| | | Let stand for 2 hours | |
| Evaluation of Tamerit stability in Fed state simulating intestinal fluid (FeSSIF). | 500ml apparatus II 37°C | FeSSIF pH 5.0 + Add 450mg Pancreatin to each vessel | **30min** |
| | | | **60min** |
| | | | **120min** |
| | In triplicate 75 RPM | | **180min** |
| | Tamerit amount is 100 mg in each unit | (preparation of 2L of media: 8.08g of NaOH pellets, 17.3g AcAc glacial, 23.75g NaCl 1. 8Lwater, adjust pH to 5 with or HCl(1N) or NaOH (1N) and make up to volume 2L | |
| | | Take 22.4g of SIF powder, dissolve in 1500mL of buffer and make up to volume 2L at RT when solution is clear) | |
| Evaluation of Tamerit stability in simulating colonic fluid (SCoF). | 500ml apparatus II 37°C | FeSSCoF pH 6.0 + Lactobacilli 100Million (5drp) | **0h** |
| | | | **2h** |
| | | | **4h** |
| | In triplicate 75 RPM | | **6h** |
| | | preparation of 2L of media 1.32 g of Sodium hydroxide pellets (NaOH) 7 g of Maleic acid (C4H4O4) 7.38 g of Tris base (C4H11NO3) 1. 8Lwater, adjust pH to 6.0 with or HCl(1N) or NaOH (1N) and make up to volume 2L | **24h** |
| | Tamerit amount is 100 mg in each unit | | |
| | | Take 1.48g of FeSSCoF powder, dissolve in 1500mL of buffer and make up to volume 2L | |
| | | Let stand for 1 hour | |

### Permeation experiment procedures

### Porcine stomach permeability procedure

- Full thickness pig GI sample delivered to Solubest on the day of the experiment.
- Experiments were carried out on 6 slices 2cm*2cm with full thickness pig stomach sample according to the protocol (see figure below for the sampled tissues).
- The receiver compartment contains 0.01M Phosphate Buffer pH 7.4 (PBS).
- Receiver's samples were withdrawn at 6 time points (0, 15min, 30min, 45min, 90min and 2 hours).
- Water pump was set to temp 38±1°C in order to keep fixed temp in receptor department at 37±1°C.

### Porcine small intestinal (SI) permeability procedure

- Full thickness pig small intestinal delivered to Solubest on the day of the experiment.
- Experiments were carried out on 6 slices 2cm*2cm with full thickness pig small intestinal sample
- The intestinal was prepared so brush border side is facing the donor compartment.
- According to Solubest protocol the excess mucus and connective tissue were removed
- The receiver compartment contains 0.01M Phosphate Buffer pH 7.4 (PBS).
- Receiver's samples were withdrawn at 5 time points (30min, 60min, 120min, 4 hours and 6 hours).
- Water pump was set to temp 38±1°C in order to keep fixed temp in receptor department at 37±1°C.

### Porcine large intestinal (LI) permeability procedure

- Full thickness pig large intestine was delivered to Solubest on the day of the experiment.
- Experiments were carried out on 6 slices 2cm*2cm with full thickness pig large intestinal sample
- According to Solubest protocol the excess mucus and connective tissue was removed
- The receiver compartment contains 0.01M Phosphate Buffer pH 7.4 (PBS).
- Receiver's samples were withdrawn at 7 time-points (30min, 60min, 120min, 4 hours and 6 hours, 12 hours and 24 hours).
- Water pump was set to temp 38±1°C in order to keep fixed temp in receptor department at 37±1°C.

### Full thickness pig large intestine.

The intestine was prepared so the mucous side will be facing the donor compartment.

### Results

### Tamerit Stability in Gastric and Intestinal Fluids

Tamerit dissolution and stability observed in Gastric Fast and Fed fluids are summarized below. Each presented dissolution data is a summary of 3 results.

### Tamerit dissolution in FaSSGF pH 1.6

| ***Time, min*** | ***Dissolution, %*** |
|---|---|
| 15 | 88.24 |
| 30 | 90.68 |

### Tamerit dissolution in FeSSGF pH5.0

| ***Time, min*** | ***Dissolution, %*** |
|---|---|
| 15 | 86.83 |
| 30 | 77.65 |
| 45 | 75.6 |
| 90 | 79.42 |

### Conclusion

As can be seen from the above study on determining the stability of Tamerit on the model of the sample gastrointestinal tract of the pig, Tamerit showed sufficient stability in the fast and fed gastric conditions. The dissolution in the fed media is 80-90% and in the fast ~90%.

### Tamerit dissolution in FaSSIF pH 6.5

| ***Time, min*** | ***Dissolution, %*** |
|---|---|
| 30 | 98.62 |
| 60 | 103.11 |
| 90 | 102.16 |
| 120 | 102.37 |
| 180 | 103.78 |

### Tamerit dissolution in FeSSIF pH 5.0

| ***Time, min*** | ***Dissolution, %*** |
|---|---|
| 30 | 79.65 |
| 60 | 69.28 |
| 90 | 87.40 |
| 120 | 84.48 |
| 180 | 88.50 |

As can be seen from the data above, Tamerit is a quite stable in the fast and fed conditions of the small intestinal. The dissolution in the fed media is 90% after 90 min and in the fast ~100% after 30 minutes.

Fig. 2 shows Tamerit powder dissolution. FIG. 2 shows that Tamerit is the most soluble at pH 6.5. Tamerit dissolution and stability in simulated colonic fluids are summarized below.

**Table 7 - Tamerit dissolution in FeSSCoF pH 6.0**

| Time | Dissolution % |
|---|---|
| 15 min | 90.07 |
| 2 hr | 89.21 |
| 4 hr | 102.12 |
| 24 hr | 95.77 |

As can be seen from the data above, Tamerit is very stable in the colonic conditions. The dissolution in the fed media is 90% after 15 min, 100% after 4 hr and this value didn't drop even during 24 hr.

Tamerit 10mM solution permeability was tested in four chambers, concentration in receptor compartment described below

**Table 8 - Porcine Gastric and Intestinal tissues permeability results**

| Time, min | Tamerit stomach | | | | AVG, µg/mL |
|---|---|---|---|---|---|
| 0 | 0 | 0 | 0 | 0 | **0.000** |
| 15 | 0.0578 | 0.0721 | 0.0240 | 0.0453 | **0.050** |
| 30 | 0.0508 | 0.0466 | 0.0193 | 0.0475 | **0.041** |
| 45 | 0.1121 | 0.0699 | 0.0237 | 0.0690 | **0.069** |
| 90 | 0.1108 | 0.1482 | 0.0279 | 0.0528 | **0.085** |
| 120 | 0.1229 | 0.1205 | 0.0372 | 0.1365 | **0.104** |
| | | | | | |

| | Tamerit - Small intestine | | | | AVG, mg/mL |
|---|---|---|---|---|---|
| 0 | 0 | 0 | 0 | 0 | **0.000** |
| 30 | 0.0014 | 0.0007 | 0.0015 | 0.0009 | **0.001** |
| 60 | 0.0035 | 0.0024 | 0.0035 | 0.0024 | **0.003** |
| 120 | 0.0054 | 0.0056 | 0.0091 | 0.0051 | **0.006** |
| 240 | 0.0123 | 0.0102 | 0.0166 | 0.0082 | **0.012** |
| 360 | 0.0264 | 0.0195 | 0.0287 | 0.0190 | **0.023** |

| | Tamerit - Large intestine | | | | AVG, mg/mL |
|---|---|---|---|---|---|
| 0 | 0 | 0 | 0 | 0 | **0.000** |
| 30 | 0.0021 | 0.0055 | 0.0043 | 0.0088 | **0.005** |
| 60 | 0.0030 | 0.0044 | 0.0099 | 0.0117 | **0.007** |
| 120 | 0.0171 | 0.0149 | 0.0109 | 0.0243 | **0.017** |
| 240 | 0.0306 | 0.0250 | 0.0364 | 0.0490 | **0.035** |
| 360 | 0.0316 | 0.0296 | 0.0479 | 0.0842 | **0.048** |
| 720 | 0.0996 | 0.0517 | 0.0780 | 0.1305 | **0.090** |
| 1440 | 0.1528 | 0.1474 | 0.1440 | 0.3102 | **0.189** |

Propranolol 10mM solution (positive control) permeability was tested in two chambers, concentration in receptor compartment described below:

**Table 9 - Propranolol 10mM solution (positive control) Permeability**

| Time, min | Propranolol stomach | | AVG, mg/mL |
|---|---|---|---|
| 0 | 0 | 0 | **0.000** |
| 30 | 0.0000 | 0.0000 | **0.000** |
| 60 | 0.0000 | 0.0000 | **0.000** |
| 120 | 0.0000 | 0.0000 | **0.000** |
| 240 | 0.0000 | 0.0000 | **0.000** |
| 420 | 0.0000 | 0.0000 | **0.000** |

| Time, min | Propranolol Small intestine | | AVG, mg/mL |
|---|---|---|---|
| 0 | 0 | 0 | **0.000** |
| 30 | 0.0002 | 0.0015 | **0.001** |
| 60 | 0.0017 | 0.0074 | **0.005** |
| 120 | 0.0065 | 0.0186 | **0.013** |
| 240 | 0.0196 | 0.0460 | **0.033** |
| 360 | 0.0278 | 0.0819 | **0.055** |

**Table 10 - Propranolol**

| **Large intestine (colon) results** | | | |
|---|---|---|---|
| Time, min | Propranolol Large intestine | | AVG, mg/mL |
| 0 | 0 | 0 | **0.000** |
| 30 | 0.0019 | 0.0023 | **0.002** |
| 60 | 0.0053 | 0.0050 | **0.005** |
| 120 | 0.0108 | 0.0162 | **0.014** |
| 240 | 0.0320 | 0.0295 | **0.031** |
| 360 | 0.0379 | 0.0513 | **0.045** |
| 720 | 0.0884 | 0.0799 | **0.084** |
| 1440 | 0.1444 | 0.1343 | **0.139** |

Fig. 3 shows porcine Stomach Tamerit Permeability Results. Fig. 4 shows porcine stomach Propranolol permeability. Fig. 3 and Fig. 4 show that Tamerit and Propranolol poorly penetrate through the stomach tissue.

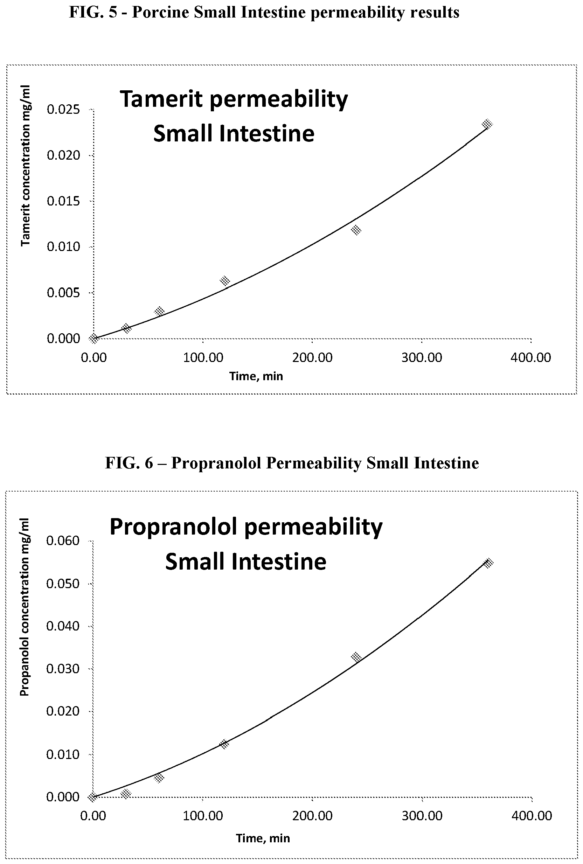

Fig. 5 shows porcine small intestine Tamerit permeability results. Fig. 5 shows that good Tamerit adsorption through the small intestine.

Fig. 6 shows porcine small intestine Propranolol permeability results. Fig. 6 shows that Propranolol is well adsorbed through the small intestine.

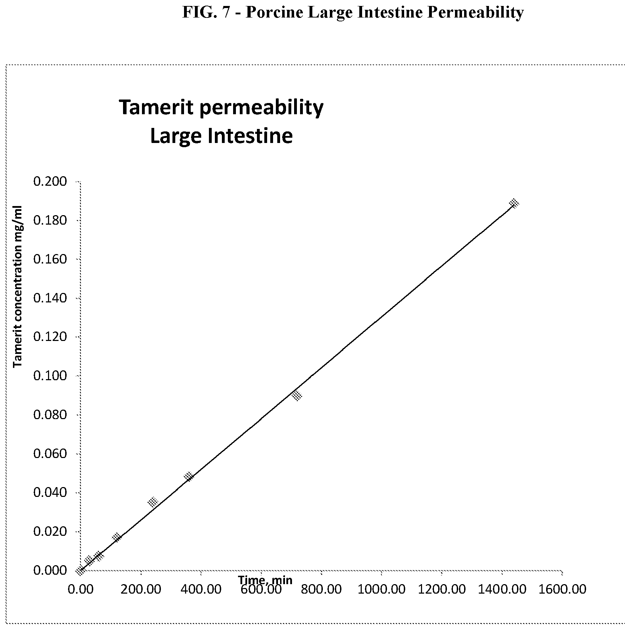

Fig. 7 shows Tamerit Porcine Large Intestine Permeability. The study showed that Tamerit has good permeability through the pig's large intestine.

**Table 11- Porcine large intestine permeability of Tamerit**

| Table 11 shows good permeability of Tamerit on the large intestine tissue model | | | |
|---|---|---|---|
| Time, min | Tamerit Concentration in receptor comp AVG, mg/mL | mg in receptor comp | Recovery from donor comp, % |
| 0 | **0.000** | **0.000** | 0.00 |
| 30 | **0.005** | **0.026** | 0.45 |
| 60 | **0.007** | **0.036** | 0.63 |
| 120 | **0.017** | **0.084** | 1.46 |
| 240 | **0.035** | **0.176** | 3.07 |
| 360 | **0.048** | **0.242** | 4.20 |

**Table 12-Propranolol permeability, large intestine**

| | | | |
|---|---|---|---|
| 0 | **0.000** | **0.000** | 0.00 |
| 30 | **0.002** | **0.010** | 0.16 |
| 60 | **0.005** | **0.026** | 0.40 |
| 120 | **0.014** | **0.068** | 1.04 |
| 240 | **0.031** | **0.154** | 2.36 |
| 360 | **0.045** | **0.223** | 3.43 |
| 720 | **0.084** | **0.421** | 6.47 |
| 1440 | **0.139** | **0.697** | 10.72 |

Table 12 shows that propranolol is well adsorbed in the large intestine.

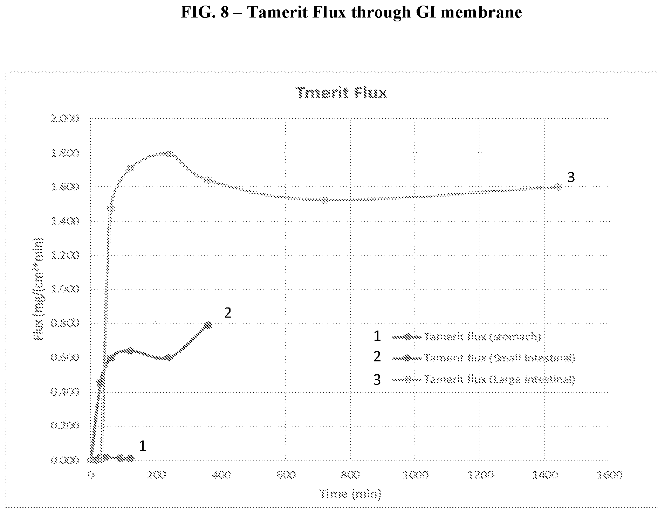

Fig. 8 shows Tamerit Flux through GI membrane. The study showed that Tamerit has good permeability through the large and small intestine tissues and less actively permeates through stomach tissue.

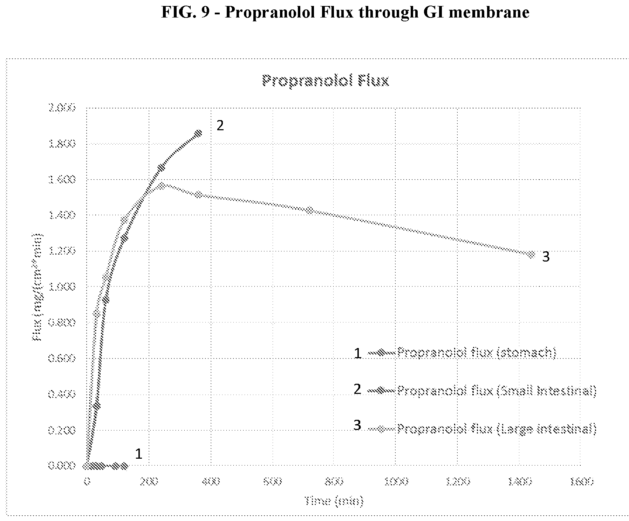

Fig. 9 shows Propranolol Flux through GI membrane. The study showed that propranolol permeates well through the large and small intestine tissues.

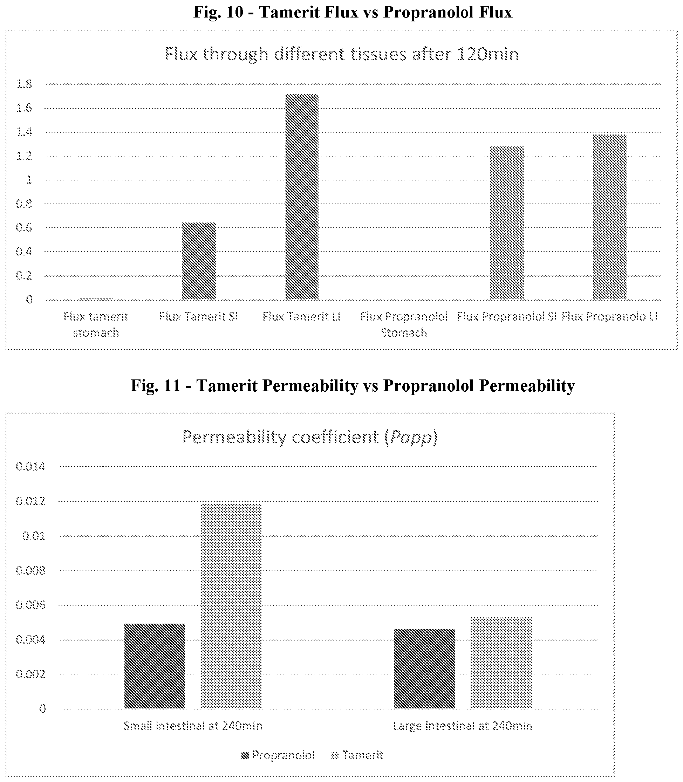

Fig. 10 shows Tamerit Flux vs Propranolol Flux through stomach, small and large intestine after 120 min.

Fig. 11 shows Tamerit Permeability vs Propranolol Permeability. The study showed that Tamerit permeates better than propranolol through small and large intestine.

### Conclusions

- Both ADPS and Propranolol demonstrated almost no permeation through gastric tissues
- ADPS has good permeability through small and large intestinal tissues
- ADPS flux characteristic resembles Propranolol flux which may indicate that ADPS has high permeability. The passive transcellular transport should be considered
- ADPS presents good permeability through large intestinal tissue over long period of time reaching steady state after four hours
- Permeation of ADPS after 4 hours three time better than Propranolol in small intestinal and as good as Propranolol in large intestinal
- It can be hypothesized that ADPS absorption via oral route will be as good as Propranolol absorption.

Thus, the carried out comparative studies in comparison with propranolol, as to the degree of possibility of Tamerit's penetration through biological membranes, its active permeation through the various departments of gastro-intestinal tract, showed a high permeability and adsorption through the various tissues of biological membranes. The data received coincides with the results of the research on the pharmacokinetics of the drug. These results open wide opportunities for the oral application of Tamerit in clinical practice.

### EXAMPLE 5- EFFECTIVENESS OF TAMERIT IN THE TREATMENT OF NON-VIRAL HEPATITIS CAUSED BY VARIOUS PATHOLOGICAL DETERMINANTS

### A. The effect of the phthalhydrazide derivative in the treatment of acute hepatitis caused by the administration of the hepatotropic poison - carbon tetrachloride

Acute experimental hepatitis that develops with the introduction of tetrachloromethane (CCL₄) is a classic model for studying the mechanisms of reparative regeneration of the liver after exposure to various chemical toxicants.

The experiments were carried out on male Wistar rats weighing 200-220 g, in accordance with the recommendations of the international ethics committees on the humane treatment of laboratory animals (EU Council Directive 86/609 EEC dated 11/24/1986). Carbon tetrachloride (CCL₄), was administered to the animals intraperitoneally once at a dose of 50 mg/kg of body weight. Tamerit was injected to experimental animals throughout the experiment intramuscularly at the rate of 2 mg/kg, control rats were injected an equal dose of saline. The animals were sacrificed on the 3rd, 7th day after the simulation of toxic hepatitis.

Against the background of the activation of macrophages, with the use of Tamerit, as early as on the 3rd day of the experiment, the signs of toxic hepatitis in the liver of rats are less pronounced, there is a visual increase in the number of mitotically dividing cells, signs of inflammation are reduced, polymorphonuclear leukocytes in infiltrates are rare, and by the 7th day, focal necroses without a perifocal cell reaction are only on the periphery of the lobule. Hepatocyte granularity is preserved, hepatocytes with hydropic degeneration are not detected. Morphometric analysis showed that, against the background of a high alteration index, on the 3rd day after the administration of CCL₄ and treatment with Tamerit, the processes of both cellular and intracellular regeneration of hepatocytes are more intensive in the animals. The mitotic index and the number of binuclear cells in the liver tissue increase compared with the control animals. By the 7th day of the experiment, these parameters decrease, significantly exceeding the corresponding values observed in intact healthy rats.

It is known that a necessary condition for the completion of a regenerative process is, above all, the restoration of the functional status of the damaged tissue. The blood biochemical parameters in the development of toxic hepatitis was studied, allowing to determine the relationship between liver regeneration and its functional state and the role of macrophages in the correlation of these two processes.

**Table 13 - Blood biochemical parameters of experimental animals with acute hepatitis and against the background of treatment with Tamerit**

| Parameter | Intact | CCl₄ 3rd day | CCl₄ 3rd day + Tamerit | CCl₄ 7th day | CCl₄ 7th day + Tamerit |
|---|---|---|---|---|---|
| AST, U/L | 10.4 ±1.1 | 14.3±2.5 | 10.0±2.0 | 10.64±0.85 | 8.53±0.13** |
| ALT, U/L | 6.0±0.9 | 14.3±3.2* | 9.1±1.6* | 12.36± 0.79* | 7.12±0.20** |
| BCL, quant | 210.0±2.0 | 277.0±30.0* | 186.0±7.0 *^{,}** | 287.2±17.17* | 171±18*^{,}** |

| | | | | | |
|---|---|---|---|---|---|
| Note * - the differences with the group of intact animals are significant with P<0.05; ** - the differences with the group that received CCl₄ are significant with P<0.05. | | | | | |

As a result of the study, it can be concluded that in the early stages of the development of toxic hepatitis caused by the administration of tetrachloromethane, the administration of Tamerit reduces the severity of the inflammatory reaction in response to damage of hepatocytes under the action of the toxicant, enhances regenerative processes in the liver manifested in an increase in the number of binuclear and mitotic cells. At the same time, the parameters of endogenous intoxication, the intensity of free radical oxidation and the activity of cytolytic enzymes in the blood are significantly reduced.

### B. The effect of Tamerit in the treatment of hepatitis caused by pesticides

A derivative of 2,4-dichlorophenoxyacetic acid (2,4-D) and its salts, such as the amine salt, is one of the most widely used pesticides in agriculture. Literary data indicate the damaging effect of pesticides on internal organs, in particular the liver, pancreas, and blood-forming organs (Sh.N. Galimov, 2000, B. Rosso et al., 1997).

A 48 years old patient visited a doctor with complaints of weakness, malaise, recurrent epigastric pains on the right, shortness of breath when walking fast, rapid fatigability, dizziness. Physical examination revealed skin pallor, icteric sclera, pallor of the nail phalanx. No rash. On auscultation, heart sounds are muffled, rhythmic, satisfactory volume and tension. In the lungs - harsh breathing, no wheezing. Reduced breathing volume on exertion. On palpation, the abdomen is tense, painful in the epigastric region, both on the left and on the right. On palpation, the liver is enlarged, painful. Laboratory tests: leukocytosis (12 thousand), AST-80, ALT-98, total bilirubin -32, Hb -89, erythropenia, decreased hematocrit, increased malondialdehyde are noted. Ultrasound investigation revealed an increase in the liver by 3 cm. from under the edge of the costal arch, in the right lobe there are hyperechoic regions, the structure is heterogeneous, hepatic congestion, gallbladder volvulus, reactive pancreatitis, swollen intestines. The spleen is enlarged, heterogeneous, pronounced vascular response. Taking into account the contact with the pesticide for a long time, the patient neglected the rules of protection from herbicides, which resulted in gradual poisoning with a toxicant. After the detoxification therapy was administered to the patient, Tamerit was added to the therapy - two enteric-coated tablets containing 100 mg. of active substance twice a day for two weeks, and then one tablet for two weeks, and then one tablet once a day for two weeks, then one tablet every third day for three weeks. On the third day from the start of treatment, the patient's condition improved, weakness, malaise decreased, the skin turned pink, nausea disappeared, epigastric pain decreased. In the second week of treatment, transaminases decreased, hemoglobin and erythrocyte levels increased. After the end of treatment, the patient's condition is satisfactory without complaints. At discharge, the skin is pink, no icteritiousness. The weakness has gone, shortness of breath on exertion. Hemoglobin 120. Levels of leukocytes, erythrocytes, hematocrit normalized. Ultrasound investigation revealed that the liver borders are within the normal range, the liver is homogeneous, no pathological changes were noted, AST and ALT within normal limits. The pancreas has normal in size, ultrasound investigation revealed no hyperechoic/hypoechoic areas. The analysis results are normal. Malondialdehyde levels are at healthy levels. Bilirubin is normal. The spleen is not enlarged, the structure is normal. No deviations in the bronchopulmonary system. Cardiovascular system without pathology. The patient was discharged from the hospital in satisfactory condition. In this example, it was found that cumulative exposure to the pesticide 2,4-D results in damage of organs, especially the liver, pancreas, spleen and red blood cells, accompanied by a decrease in the number of erythrocytes, hemoglobin, hematocrit in peripheral blood. Clinically, this is manifested by pallor, fatigue, shortness of breath on exertion, hypoxia of the organs, respectively, and of the organism as a whole. The lipid peroxidation that develops at the same time causes tissue damage, and in response, macrophages infiltrate and generate oxygen radicals and pro-inflammatory cytokines (TNF, IL, etc.), thereby contributing their destructive potential to the development of the pathological process. By reducing the degree of peroxidation Tamerit protects the tissue from damage, and prevents the infiltration of neutrophilic granulocytes, preventing further development of the pathological process in the body in response to the toxic effects of 2,4-D. It was noted that the dose of the drug is prescribed depending on the dose of the toxicant and the sooner Tamerit is prescribed, the sooner the therapeutic effect can be obtained.

### C. The effect of Tamerit in the treatment of acute hepatitis caused by the introduction of alcohol substitutes

Periodically, in various countries, severe poisonings with surrogate drinks, including alcohol solutions of polyhexamethyleneguanidine (PHMG), intended for disinfection, are recorded. What draws attention is the "selectivity" of the poison effect, possibly associated with a certain predisposition of some patients, for example, through the presence of chronic alcoholic hepatitis, and, consequently, the development of fulminant liver failure and renal failure.

The experiment was carried out on 25 male white outbred rats weighing 200-300 g, in accordance with the recommendations of the international ethics committees on the humane treatment of laboratory animals (EU Council Directive 86/609 EEC dated 11/24/1986). To create an experimental model, an aqueous solution of PHMG was used, which was administered intraperitoneally at a dose of 50 mg/100 g. Since the mortality from a single injection of PHMG was about 70%, the drug was administered fractionally (17 mg/100 g) during the day. All animals were divided into 5 experimental groups: group 1 - intact animals, group 2 - PHMG poisoning (3 days), group 3 - PHMG poisoning (7 days), group 4 - PHMG poisoning (3 days) + administration of Tamerit, group 5 - PHMG poisoning (7 days) + administration of Tamerit. Tamerit was administered intraperitoneally by dissolving the active substance 50 mg. per 100g.

By the 7th day of the experiment with the introduction of PHMG, destructive-alternative processes in the liver of experimental animals with a mild inflammatory reaction increased. Signs of thrombus formation were found in part of the veins of the portal tracts. Against the background of the modulation of macrophage activity by Tamerit, the degree of hepatocyte destruction was minimal, but focal accumulations of lymphocytes and macrophages were found in the organ parenchyma. Morphometric studies showed that the mitotic index of hepatocytes after the introduction of PHMG against the background of the administration of Tamerit was significantly higher than the control values on the 7th day after the introduction of the toxicant. Expression of the Ki-67 marker by hepatocytes during treatment increased both on day 7 and day 7, which proves the potential ability of liver cells to divide. Counting the number of binuclear cells showed their increase on the 3rd and 7th day of the experiment compared to intact animals in all study groups (Table 13).

**Table 14 - The effect of PHMG toxicity on parameters of cellular and intracellular regeneration of hepatocytes**

| Parameter s | Intact | PHMG - 3 days | PHMG - 3 days Tamerit | PHMG - 7 days | PHMG - 7 days Tamerit |
|---|---|---|---|---|---|
| Mitotic index, % | 0.52±0.3 3 | 39.68± 0.22* | 38.26± 0.18* | 40.26±0.16* | 45.6±0.14* ** |
| Ki-67 positive cells | 14.3±1.5 | 5.85± 2.5* | 66.3± 46.8* | 32.0±9.2*^{,}** | 96.7±9.0* |
| Binuclear cells, % | 15.4±1.4 3 | 96.93±0.33 * | 65.12±0.14 * | 76.82±0.18*^{,}* * | 61.18±0.24 * |

| | | | | | |
|---|---|---|---|---|---|
| Note * - the differences with the group of intact animals are significant with P<0.05; ** - the differences with the group that received PHMG are significant with P<0.05. | | | | | |

Treatment with Tamerit also contributed to a decrease in the activity of cytolytic enzymes in the blood of experimental animals (Table 15).

**Table 15 - Blood biochemical parameters of experimental animals after exposure to PHMG and against the background of treatment with Tamerit**

| Parameter | Intact | PHMG 3rd day | PHMG 3rd day + Tamerit | PHMG 7th day | PHMG 7th day + Tamerit |
|---|---|---|---|---|---|
| AST, U/L | 10.8 ±1.0 | 10.4±0.2 | 11.3±0.6 | 15.4±0.6* | 12.1±0.3*^{,}** |
| ALT, U/L | 6.0±0.9 | 5.8±0.8 | 5.7±1.9 | 12.8±1.0* | 8.1±0.2*^{,}** |
| GTP, U/L | 25.4±3.5 | 25.7±4.1 | 17.5±2.0*^{,}** | 55.4±15.8* | 26.0±3.4*^{,}** |

| | | | | | |
|---|---|---|---|---|---|
| Note * - the differences with the group of intact animals are significant with P<0.05; ** - the differences with the group that received PHMG are significant with P<0.05. | | | | | |

Thus, the possibility of using Tamerit as a means of detoxification therapy under the action of PHMG, the toxic effect of which is largely due to damage to the structures of the vascular wall, which leads to the development of ischemia and secondary lesions that develop in the liver and kidneys, was proved. In the liver, Tamerit stabilizes the hepatocyte membranes, promotes the development of the processes of cellular and intracellular regeneration of the liver.

### D. The action of Tamerit for the treatment of acute hepatitis caused by the introduction of Acetaminophen (Paracetamol)

Paracetamol (Acetaminophen) is one of the most popular drugs in medical practice, which is actively advertised as the safest and most effective drug.

However, studies in recent years have shown that, at therapeutic dosages, Acetaminophen has a relatively low toxicity, but conscious or accidental intake of high doses of the drug can lead to severe damage to the liver, often resulting in the death of the patient.

The experiment was carried out on 45 adult male outbred white rats weighing 288.5±12.9 g, in accordance with the recommendations of the international ethics committees on the humane treatment of laboratory animals (EU Council Directive 86/609 EEC dated 11/24/1986). To create an experimental model, an aqueous solution of Acetaminophen was administered intragastrically using a probe at a dosage of 110 mg/kg of body weight, which is about 2 times higher than the maximum allowable daily dose in humans. The animals were divided into 9 experimental groups: the first group is intact animals; the second, third, fourth and fifth groups of the animals were administered only Acetaminophen; the fifth, sixth, seventh, eighth, ninth experimental groups were treated with Tamerit. The drug was administered intragastrically, using a probe, in the form of an aqueous solution

Based on the studies conducted in the animal experiment, the toxic effects of Acetaminophen in dynamics were evaluated. It was established that with a single injection of the drug toxic hepatitis develops in all rats. The toxic effect of Paracetamol is expressed in focal necrosis of hepatocytes, as well as in disorder of the circulatory system of the liver even in the early stages of the experiment and remains stable for 12 days. The fact of hepatocyte necrosis is proved not only by morphological data, but also by significant changes in blood biochemical parameters, such as ALT, AST and GTP. The growth of free-radical processes is observed only on the 5th and 7th day, and by the 12th day the severity of endogenous intoxication decreases.

In the rats treated with the Tamerit, the signs of toxic hepatitis are less pronounced, the number of necrotic hepatocytes is much less than in the liver of rats that were not treated. On the 7th and 12th day of treatment, necrosis remains only along the periphery of the lobule, the inflammatory response is reduced. Mitoses of hepatocytes and an increase in the number of binuclear cells are clearly visible. There is a decrease in free-radical oxidation, for instance, the biochemiluminescence (BCL) of blood plasma decreases in all the studied periods. The activity of cytolytic enzymes in the blood decreases. This indicates stabilization of the hepatocyte membrane, normalization of their functional state (Tables 16 and 17).

**Table 16 - Biochemiluminescence of blood plasma (BCL, quant)**

| Groups | Study dates | | | |
|---|---|---|---|---|
| | 3 days | 5 days | 7 days | 12 days |
| Intact animals 210±2.0 | | | | |
| Acetaminophen | 220.0±17.0 | 241.0±28.0* | 257.0±16.0* | 227±22.0* |
| Acetaminophen + Tamerit | 213.0± 4.0 | 192.8.0±7.0*^{,}** | 154.2.0±18*^{,}** | 215.6+5.0** |

| | | | | |
|---|---|---|---|---|
| Note * - the differences with the group of intact animals are significant with P<0.05; ** - the differences with the group that received Acetaminophen are significant with P<0.05. | | | | |

**Table 17 - The activity of cytolytic blood enzymes in rats with the introduction of Acetaminophen and during treatment with Tamerit**

| Groups | AST, intact 10.8±1.0 U/L | ALT, intact 6.0±0.9 U/L | AST/ALT | GTP, intact 25.4±3.5 U/L |
|---|---|---|---|---|
| Introduction of Acetaminophen | | | | |
| 3rd day | 32.4±1.7* | 27.7±1.0* | 0.94±0.03* | 46.3±0.5* |
| 5th day | 18.0±0.2* | 19.4±1.0* | 0.94±0.03* | 44.2±0.9* |
| 7th day | 22.2±0.9* | 17.7±1.1* | 1.26±0.06* | 45.1±2.1* |
| 12th day | 28.9±1.4* | 24.7±0.9* | 1.18±0.10* | 46.6±1.2* |

| Introduction of Acetaminophen + Tamerit | | | | |
|---|---|---|---|---|
| 3rd day | 23.61±1.2*^{,}** | 17.45±1.3*^{,}** | 1.35± 0.03*^{,}** | 35.8±1.2*^{,}** |
| 5th day | 15.0±1.0*^{,}** | 12.2±0.9*^{,}** | 1.24±0.01*^{,}** | 33.8±0.9*^{,}** |
| 7th day | 16.01±2.0*^{,}** | 12.0±0.8*^{,}** | 1.33±0.02*^{,}** | 30.8±1.8*^{,}** |
| 12th day | 22.1 ±1.5*^{,}** | 18.0±1.8*^{,}** | 1.22±0.04*^{,}** | 29.4±1.5*^{,}** |

| | | | | |
|---|---|---|---|---|
| Note * - the differences with the group of intact animals are significant with P<0.05; ** - the differences with the group that received Acetaminophen are significant with P<0.05. | | | | |

Thus, the possibility of using Tamerit as a means of detoxification therapy for drug-induced hepatitis caused by the introduction of Acetaminophen was proved. In the liver, Tamerit stabilizes the hepatocyte membranes, reduces the activity of cytolytic enzymes in the blood, reduces free radical oxidation, promotes the development of the processes of cellular and intracellular regeneration of the liver.

### E. The effect of Tamerit in the treatment of hepatitis caused by the introduction of food poisons (carrageenan)

The experiments were carried out on 25 male Wistar rats weighing 200-220 g, in accordance with the recommendations of the international ethics committees on the humane treatment of laboratory animals (EU Council Directive 86/609 EEC dated 11/24/1986). Carrageenan is a widely known polymer of polygalactose, a drug used in the food industry that has toxicity to hepatocytes of the liver. Its mechanism of action is based on the inhibition of the process of phagocytosis and the activity of lysosomal hydrolases of mononuclear phagocytes. The drug was administered intragastrically through a probe in an aqueous solution at a dose of 10 mg/kg. The choice of time frame (4, 17 hours after surgery) corresponds to the phases of liver regeneration. All animals were divided into 5 experimental groups: group 1 - intact animals, group 2 - introduction of carrageenan (4 hours), group 3 - introduction of carrageenan (17 hours), group 4 - introduction of carrageenan (4 hours) + administration of Tamerit, group 5 - introduction of carrageenan (17 hours) + administration of Tamerit.

Morphological analysis showed that the introduction of carrageenan to healthy intact rats does not cause obvious pathological changes in the liver after 4 hours and 17 hours. Morphological picture of the liver corresponds to healthy intact animals. However, morphometric analysis data show that carrageenan reduces intracellular regeneration rates and completely blocks cellular regeneration of the liver. The mitotic index of hepatocytes in all studied periods was zero.

With the administration of Tamerit, the number of binuclear hepatocytes increases, thus returning their mitosis ability.

**Table 18 - Histological parameters of the liver of intact rats that received carrageenan and against the background of the administration of Tamerit**

| Parameters | Intact | Carrageenan 4 hours | Carrageenan 17 hours | Carrageenan 4 hours+ Tamerit | Carrageenan 17 hours+ Tamerit |
|---|---|---|---|---|---|
| Number of binuclear cells, % | 15.40±1.43 | 19.00±1.14 | 20.80±2.38 | 25.0±3.58* | 29.2±4.18* |
| Nuclear cytoplasmic index | 0.270±0.006 | 0.35±0.02* | 0.307±0.003* | 0.36±0.01* | 0.35±0.02* |
| Mitotic index, % | 0.52±0.33 | 0 | 0 | 0.65±0.25** | 0.48±0.12** |

| | | | | | |
|---|---|---|---|---|---|
| Note * - the differences with the group of intact animals are significant with P<0.05; ** - the differences with the group that received carrageenan are significant with P<0.05. | | | | | |

The introduction of carrageenan contributes to a persistent increase in AST activity in both study periods; ALT activity, on the contrary, decreases significantly 17 hours after the injection, which indicates a decrease in metabolic processes in hepatocytes. Treatment with Tamerit completely restores the activity of aminotransferases to the parameters of healthy animals.

**Table 19 - Blood aminotransferase activity with the introduction of carrageenan and against the background of treatment with Tamerit**

| Parameter | Intact | Introduction of carrageenan | | Introduction of carrageenan+ Tamerit | |
|---|---|---|---|---|---|
| | | 4 hours | 17 hours | 4 hours | 17 hours |
| ALT, units/l | 38.05±5.30 | 47.40±13.37 | 19.50±2.60* | 40.2±4.0 | 42.2±3.5 |
| AST, units/l | 87.67±12.90 | 150.2 ± 13.4* | 251.2 ± 27.7* | 85.4±8.0 | 83.5±4.5 |

| | | | | | |
|---|---|---|---|---|---|
| Note * - the differences with the group of intact animals are significant with P<0.05; ** - the differences with the group that received carrageenan are significant with P<0.05. | | | | | |

Thus, Tamerit can be used in the treatment of acute hepatitis caused by the introduction of food poison.

### F. The effect of Tamerit in the treatment of traumatic liver damage caused by partial hepatectomy

The experiments were carried out on 25 Wistar male rats weighing 200-220 g, in accordance with the recommendations of the international ethics committees on the humane treatment of laboratory animals (EU Council Directive 86/609 EEC dated 11/24/1986). Reparative liver regeneration was caused by the removal of 2/3 mass of the organ - partial hepatectomy by G.M. Higgis, R.M. Anderson. The choice of time frame (4, 17 hours after surgery) corresponds to the phases of liver regeneration.

When macrophage activity is stimulated by Tamerit, as soon as 4 hours after the operation, an increase in hepatocyte nuclei is observed, which indicates that cells are preparing for mitosis, accompanied by activation of the synthesis of fission proteins that are involved in the regulation of the mitotic cycle and DNA replication. 17 hours after the operation, as a result of the action of Tamerit, there is an increase in the mass of the preserved part of the organ. In the regenerating liver, the activation of the macrophage component by Tamerit increases the number of hepatocytes that divide by mitosis by an order of magnitude.

**Table 20 - Histological parameters of rat liver after partial hepatectomy with the administration of Tamerit**

| | Intact | Hepatectomy 4 hours | 4 hours +Tamerit | Hepatectomy 17 hours | 17 hours+ Tamerit |
|---|---|---|---|---|---|
| The size of the nucleus of hepatocytes, µm | 6.28±0.12 | 7.23±0.27* | 8.0±0.26* | 7.67±0.88 | 7.67±0.28*^{,}** |
| Number of binuclear cells, % | 15.4±1.43 | 21.30±0.88* | 16.6±1.69 | 15.75±2.06** | 29.83±2.5*^{,}** |
| Nuclear cytoplasmic index | 0.27±0.006 | 0.344±0.008* | 0.40±0.005* | 0.348±0.008* | 0.319±0.01*^{,}** |
| Mitotic index, % | 0.52±0.33 | 0.25±0.25 | 2±0.8* | 0.25±0.25 | 5.5±1.3*^{,}** |

| | | | | | |
|---|---|---|---|---|---|
| Note * - the differences with the group of intact animals are significant with P<0.05; ** - differences with the group 4 hours after hepatectomy are significant with P<0.05. | | | | | |

Aspects of the invention relates to compositions and methods to influence the immune system by modulating the activity of macrophages, which leads to the normalization of clinical and biochemical parameters, the functional state of the liver, reduction of the inflammatory response and the severity of endogenous intoxication. In some embodiments, the duration of the treatment to complete recovery is reduced. In some embodiments, the risk of possible complications is reduced.

### EXAMPLE 6- Case study

A 55 years old patient was taken to the center for the treatment of acute poisoning with symptoms of liver failure. From the anamnesis, it was established that 3 days ago, for the purpose of alcohol intoxication, he used an unknown alcohol-containing liquid. During the last days the patient's condition worsened: nausea, repeated vomiting, and his relatives noticed yellowness of the skin. Blood test showed absence of C1-C2 alcohols, carbon tetrachloride and dichloroethane. Upon admission, the patient is diagnosed: Toxic hepatopathy degree II.

When examined, the patient's condition was serious, he was conscious, but sluggish, inhibited. Pronounced yellowness of the skin and mucosa, bleeding from the injection sites. The tongue is strawberry, dry, coated tongue. Muffled heart sounds, rhythm is regular. Blood pressure is 100/60, tachycardia is 120 per minute. When auscultating the lungs, breathing is heard over the entire surface, congestive rales in the lower sections, especially on the right. Free fluid in the abdominal cavity is not detected. Abdominal palpation showed enlarged liver, protruding from under the costal arch up to 5 cm, it is dense, painful on palpation. The area of the pancreas is painful. Upon urinary catheterization - beer-colored urine. Peristalsis is active. No stool for 2 days. Laboratory tests results: total bilirubin, direct and indirect are 256.9, 103.7 and 153.2 µmol/l respectively. ALT - 430.7 U/L. AST - 167 U/L. On the 5th-7th day, bilirubin remained almost unchanged, and the level of intracellular enzymes increased on average by 20-25%. Clinically, the condition has worsened, laboratory parameters without improvement. The patient received standard hepatic therapy: Duphalac 50 ml 3 times a day, Hepasol A 1000 ml, Heptral 800 mg. Detoxification therapy was carried out. Starting from the third day, taking into account the increase in the level of intracellular enzymes (ALT, AST), Tamerit was prescribed PER OS as an anti-inflammatory, antioxidant therapy at a dose of 100-400 mg for 3 days, and then 100 mg 3 times a day, then 1 tablet 1-2 times a day until the symptoms of intoxication disappear. For the entire course, the patient took Tamerit in the form of enteric coated tablets. The patient was discharged on the 22nd day in a relatively satisfactory condition. The patient was monitored for another 1 month in a poison center. The drug has been shown to be highly effective in relieving symptoms of intoxication. The patient's condition improved progressively, blood biochemical parameters were fully normalized. The patient was discharged from the hospital in satisfactory condition. During the use of Tamerit, no adverse effects were noted.

## Claims

1. An oral pharmaceutical composition comprising:
a. a therapeutically effective amount of aminodihydrophthalazinedione sodium (ADPS), and
b. pharmaceutically acceptable excipients,
for use in the treatment of a disease in a subject in need thereof, the treatment comprising administering orally to a subject in need thereof of a therapeutically effective dose of the pharmaceutical composition,
wherein the disease is non-viral hepatitis, drug-induced hepatitis or autoimmune hepatitis.

2. The pharmaceutical composition for use of claim 1, wherein the pharmaceutical composition is an immediate release composition, extended release composition or a combination thereof.

3. The pharmaceutical composition for use of claim 2, the composition comprising 10-1000 mg aminodihydrophthalazinedione sodium (ADPS); or the composition comprising 100-200 mg aminodihydrophthalazinedione sodium (ADPS).

4. The pharmaceutical composition for use of any of claims 1 to 3,
wherein the composition is an immediate release composition and wherein the composition comprises a core and a coating, wherein the core comprises 10-1000 mg ADPS, 100-500 mg of filler, 5-50 mg binder, 5-80 mg disintegrant, 2-30 mg buffering agent, 5-20 mg glidant, 3-10 mg lubricant and wherein the coating comprises 10-50 mg of a humidity protecting agent; or
wherein the composition is an immediate release composition and wherein the composition comprises a core and a coating, wherein the core comprises 100-200 mg ADPS, 100-250 mg of filler, 5-20 mg binder, 5-30 mg disintegrant, 5-15 mg glidant, 3-7 mg lubricant and optionally 2-20 mg buffering agent, and wherein the coating comprises 10-30 mg of a humidity protecting agent.

5. The pharmaceutical composition for use of any of claims 1 to 3, wherein the composition is an extended release composition, wherein the composition comprises a core and optionally a coating, wherein the core comprises 10-800 mg ADPS, 100-500 mg of filler, 10-50 mg buffering agent, 50-500 mg of matrix agent, 5-20 mg glidant and 3-10 mg lubricant and optionally 10-30 mg buffering agent, and wherein the optional coating comprises 10-30 mg of a humidity protecting coating agent; or
wherein the composition is an extended release composition, wherein the composition comprises a core and optionally a coating, wherein the core comprises 100-200 mg ADPS, 100-400 mg of filler, 5-20 mg binder, 50-200 of matrix agent, 5-30 mg disintegrant, 5-15 mg glidant, 3-7 mg lubricant and optionally 10-30 mg buffering agent and wherein the optional coating comprises 10-30 mg of a humidity protecting coating agent.

6. The pharmaceutical composition for use of claim 4 or 5, wherein the buffering agent maintains neutral pH and enables proper solubility of ADPS,
wherein the buffering agent is sodium carbonate; and/or
wherein the filler is selected from mannitol, microcrystalline cellulose (Avicel 102), lactose for direct compression, dextrose, sorbitol, mannitol, maltitol, xylitol and any combination thereof; and/or
wherein the binder is selected from povidone (PVP K25), maize starch, cellulose ethers (Methocell, Ethocel), Mg stearate, gelatin, sucrose and any combination thereof; and/or
wherein the disintegrant is selected from PVP, dry potato starch, sodium starch glycolate, microcrystalline cellulose, magnesium stearate and any combination thereof; and/or
wherein the glidant is selected from magnesium stearate, fumed silica, starch, talc and any combination thereof; and/or
wherein the lubricant is selected from Mg stearate, talc, silica, fats, stearin, stearic acid and any combination thereof.

7. The pharmaceutical composition for use of any of the preceding claims, wherein the ADPS is in a form of anhydrous ADPS.

8. The pharmaceutical composition for use of any of claims 1 to 6, wherein the ADPS is in a form of ADPS dihydrate.

9. The pharmaceutical composition for use of any of claims 1 to 8, wherein the ADPS is administered in combination with a therapeutic agent.

10. The pharmaceutical composition for use of any of claims 1 to 9, wherein the step of administering comprises administering a single dose of the pharmaceutical composition daily.

11. The pharmaceutical composition for use of claim 10, wherein the single dose of 50-800 mg is administered daily for 5-50 days.

## Patentansprüche

1. Orale pharmazeutische Zusammensetzung, umfassend:
a. eine therapeutisch wirksame Menge von Aminodihydrophthalazindion-Natrium (ADPS) und
b. pharmazeutisch annehmbare Hilfsstoffe,
zur Verwendung bei der Behandlung einer Krankheit in einem Subjekt, das dies benötigt, wobei die Behandlung die orale Verabreichung einer therapeutisch wirksamen Dosis der pharmazeutischen Zusammensetzung an ein Subjekt, das dies benötigt, umfasst,
wobei die Krankheit nicht-virale Hepatitis, medikamenteninduzierte Hepatitis oder Autoimmunhepatitis ist.

2. Pharmazeutische Zusammensetzung zur Verwendung nach Anspruch 1, wobei die pharmazeutische Zusammensetzung eine Zusammensetzung mit sofortiger Freisetzung, eine Zusammensetzung mit verlängerter Freisetzung oder eine Kombination davon ist.

3. Pharmazeutische Zusammensetzung zur Verwendung nach Anspruch 2, wobei die Zusammensetzung 10-1000 mg Aminodihydrophthalazindion-Natrium (ADPS) umfasst; oder die Zusammensetzung 100-200 mg Aminodihydrophthalazindion-Natrium (ADPS) umfasst.

4. Pharmazeutische Zusammensetzung zur Verwendung nach einem der Ansprüche 1 bis 3,
wobei die Zusammensetzung eine Zusammensetzung mit sofortiger Freisetzung ist und wobei die Zusammensetzung einen Kern und einen Überzug umfasst, wobei der Kern 10-1000 mg ADPS, 100-500 mg Füllstoff, 5-50 mg Bindemittel, 5-80 mg Sprengmittel, 2-30 mg Puffermittel, 5-20 mg Gleitmittel, 3-10 mg Schmiermittel umfasst und wobei der Überzug 10-50 mg eines Feuchtigkeitsschutzmittels umfasst; oder
wobei die Zusammensetzung eine Zusammensetzung mit sofortiger Freisetzung ist und wobei die Zusammensetzung einen Kern und einen Überzug umfasst, wobei der Kern 100-200 mg ADPS, 100-250 mg Füllstoff, 5-20 mg Bindemittel, 5-30 mg Sprengmittel, 5-15 mg Gleitmittel, 3-7 mg Schmiermittel und gegebenenfalls 2-20 mg Puffermittel umfasst und wobei der Überzug 10-30 mg eines Feuchtigkeitsschutzmittels umfasst.

5. Pharmazeutische Zusammensetzung zur Verwendung nach einem der Ansprüche 1 bis 3,
wobei die Zusammensetzung eine Zusammensetzung mit verlängerter Freisetzung ist, wobei die Zusammensetzung einen Kern und gegebenenfalls eine Beschichtung umfasst, wobei der Kern 10-800 mg ADPS, 100-500 mg Füllstoff, 10-50 mg Puffermittel, 50-500 mg Matrixmittel, 5-20 mg Gleitmittel und 3-10 mg Schmiermittel und gegebenenfalls 10-30 mg Puffermittel umfasst, und wobei die optionale Beschichtung 10-30 mg eines feuchtigkeitsschützenden Beschichtungsmittels umfasst; oder
wobei die Zusammensetzung eine Zusammensetzung mit verlängerter Freisetzung ist, wobei die Zusammensetzung einen Kern und gegebenenfalls eine Beschichtung umfasst, wobei der Kern 100-200 mg ADPS, 100-400 mg Füllstoff, 5-20 mg Bindemittel, 50-200 mg Matrixmittel, 5-30 mg Sprengstoff, 5-15 mg Gleitmittel, 3-7 mg Schmiermittel und gegebenenfalls 10-30 mg Puffermittel umfasst und wobei die optionale Beschichtung 10-30 mg eines feuchtigkeitsschützenden Beschichtungsmittels umfasst.

6. Pharmazeutische Zusammensetzung zur Verwendung nach Anspruch 4 oder 5, wobei das Puffermittel einen neutralen pH-Wert aufrechterhält und eine angemessene Löslichkeit von ADPS ermöglicht,
wobei das Puffermittel Natriumcarbonat ist; und/oder
wobei der Füllstoff ausgewählt ist aus Mannit, mikrokristalliner Cellulose (Avicel 102), Lactose für direkte Kompression, Dextrose, Sorbitol, Mannit, Maltit, Xylit und jeder Kombination davon; und/oder
wobei das Bindemittel aus Povidon (PVP K25), Maisstärke, Celluloseethern (Methocell, Ethocel), Mg-Stearat, Gelatine, Saccharose und einer beliebigen Kombination davon ausgewählt ist; und/oder
wobei das Sprengmittel ausgewählt ist aus PVP, trockener Kartoffelstärke, Natriumstärkeglykolat, mikrokristalliner Cellulose, Magnesiumstearat und einer beliebigen Kombination davon; und/oder
wobei das Gleitmittel ausgewählt ist aus Magnesiumstearat, pyrogener Kieselsäure, Stärke, Talkum und einer beliebigen Kombination davon; und/oder
wobei das Schmiermittel ausgewählt ist aus Magnesiumstearat, Talkum, Siliciumdioxid, Fetten, Stearin, Stearinsäure und einer beliebigen Kombination davon.

7. Die pharmazeutische Zusammensetzung zur Verwendung nach einem der vorhergehenden Ansprüche, wobei das ADPS in Form von wasserfreiem ADPS vorliegt.

8. Die pharmazeutische Zusammensetzung zur Verwendung nach einem der Ansprüche 1 bis 6, wobei das ADPS in Form von ADPS-Dihydrat vorliegt.

9. Pharmazeutische Zusammensetzung zur Verwendung nach einem der Ansprüche 1 bis 8, wobei das ADPS in Kombination mit einem therapeutischen Mittel verabreicht wird.

10. Pharmazeutische Zusammensetzung zur Verwendung nach einem der Ansprüche 1 bis 9, wobei der Schritt der Verabreichung die tägliche Verabreichung einer Einzeldosis der pharmazeutischen Zusammensetzung umfasst.

11. Pharmazeutische Zusammensetzung zur Verwendung nach Anspruch 10, wobei eine Einzeldosis von 50-800 mg täglich über 5-50 Tage verabreicht wird.

## Revendications

1. Composition pharmaceutique orale comprenant :
a. une quantité thérapeutiquement efficace d'aminodihydrophtalazinedione sodique (ADPS), et
b. des excipients pharmaceutiquement acceptables,
pour utilisation dans le traitement d'une maladie chez un sujet qui le nécessite, le traitement comprenant l'administration orale à un sujet qui le nécessite d'une dose thérapeutiquement efficace de la composition pharmaceutique,
dans laquelle la maladie est une hépatite non virale, une hépatite induite par un médicament ou une hépatite auto-immune.

2. Composition pharmaceutique pour utilisation selon la revendication 1, dans laquelle la composition pharmaceutique est une composition à libération immédiate, une composition à libération prolongée ou une combinaison de celles-ci.

3. Composition pharmaceutique pour utilisation selon la revendication 2, la composition comprenant 10 à 1000 mg d'aminodihydrophtalazinedione sodique (ADPS) ; ou la composition comprenant 100 à 200 mg d'aminodihydrophtalazinedione sodique (ADPS).

4. Composition pharmaceutique pour utilisation selon l'une quelconque des revendications 1 à 3,
dans laquelle la composition est une composition à libération immédiate et dans laquelle la composition comprend un noyau et un enrobage, dans laquelle le noyau comprend 10 à 1000 mg d'ADPS, 100 à 500 mg de charge, 5 à 50 mg de liant, 5 à 80 mg de délitant, 2 à 30 mg d'agent tampon, 5 à 20 mg d'agent de glissement, 3 à 10 mg de lubrifiant et dans laquelle l'enrobage comprend 10 à 50 mg d'un agent de protection contre l'humidité ; ou
dans laquelle la composition est une composition à libération immédiate et dans laquelle la composition comprend un noyau et un enrobage, dans laquelle le noyau comprend 100 à 200 mg d'ADPS, 100 à 250 mg de charge, 5 à 20 mg de liant, 5 à 30 mg de délitant, 5 à 15 mg d'agent de glissement, 3 à 7 mg de lubrifiant et facultativement 2 à 20 mg d'agent tampon, et dans laquelle l'enrobage comprend 10 à 30 mg d'un agent de protection contre l'humidité.

5. Composition pharmaceutique pour utilisation selon l'une quelconque des revendications 1 à 3, dans laquelle la composition est une composition à libération prolongée, dans laquelle la composition comprend un noyau et facultativement un enrobage, dans laquelle le noyau comprend 10 à 800 mg d'ADPS, 100 à 500 mg de charge, 10 à 50 mg d'agent tampon, 50 à 500 mg d'agent de matrice, 5 à 20 mg d'agent de glissement et 3 à 10 mg de lubrifiant et facultativement 10 à 30 mg d'agent tampon, et dans laquelle l'enrobage facultatif comprend 10 à 30 mg d'un agent d'enrobage de protection contre l'humidité ; ou
dans laquelle la composition est une composition à libération prolongée, dans laquelle la composition comprend un noyau et facultativement un enrobage, dans laquelle le noyau comprend 100 à 200 mg d'ADPS, 100 à 400 mg de charge, 5 à 20 mg de liant, 50 à 200 d'agent de matrice, 5 à 30 mg de délitant, 5 à 15 mg d'agent de glissement, 3 à 7 mg de lubrifiant et facultativement 10 à 30 mg d'agent tampon et dans laquelle l'enrobage facultatif comprend 10 à 30 mg d'un agent d'enrobage de protection contre l'humidité.

6. Composition pharmaceutique pour utilisation selon la revendication 4 ou la revendication 5, dans laquelle l'agent tampon maintient un pH neutre et permet une solubilité correcte de l'ADPS,
dans laquelle l'agent tampon est du carbonate de sodium ; et/ou
dans laquelle la charge est sélectionnée parmi le mannitol, la cellulose microcristalline (Avicel 102), le lactose pour la compression directe, le dextrose, le sorbitol, le mannitol, le maltitol, le xylitol et toute combinaison de ceux-ci ; et/ou
dans laquelle le liant est sélectionné parmi la povidone (PVP K25), l'amidon de maïs, les éthers de cellulose (Methocell, Ethocel), le stéarate de Mg, la gélatine, le saccharose et toute combinaison de ceux-ci ; et/ou
dans laquelle le délitant est sélectionné parmi la PVP, l'amidon sec de pomme de terre, le glycolate d'amidon sodique, la cellulose microcristalline, le stéarate de magnésium et toute combinaison de ceux-ci ; et/ou
dans laquelle l'agent de glissement est sélectionné parmi le stéarate de magnésium, la silice pyrogénée, l'amidon, le talc et toute combinaison de ceux-ci ; et/ou
dans laquelle le lubrifiant est sélectionné parmi le stéarate de Mg, le talc, la silice, les graisses, la stéarine, l'acide stéarique et toute combinaison de ceux-ci.

7. Composition pharmaceutique pour utilisation selon l'une quelconque des revendications précédentes, dans laquelle l'ADPS est sous forme d'ADPS anhydre.

8. Composition pharmaceutique pour utilisation selon l'une quelconque des revendications 1 à 6, dans laquelle l'ADPS est sous forme d'ADPS dihydratée.

9. Composition pharmaceutique pour utilisation selon l'une quelconque des revendications 1 à 8, dans laquelle l'ADPS est administrée en combinaison avec un agent thérapeutique.

10. Composition pharmaceutique pour utilisation selon l'une quelconque des revendications 1 à 9, dans laquelle l'étape d'administration comprend l'administration quotidienne d'une dose unique de la composition pharmaceutique.

11. Composition pharmaceutique pour utilisation selon la revendication 10, dans laquelle la dose unique de 50 à 800 mg est administrée quotidiennement pendant 5 à 50 jours.
